(19) Europäisches Patentamt
European Patent Office
Office européen des brevets

(11) EP 3 712 260 A1

(12) EUROPEAN PATENT APPLICATION
published in accordance with Art. 153(4) EPC

(43) Date of publication:
**23.09.2020 Bulletin 2020/39**

(21) Application number: **18877609.0**

(22) Date of filing: **13.11.2018**

(51) Int Cl.:
***C12N 5/22*** *(2006.01)* ***A61K 39/00*** *(2006.01)*
***A61P 31/00*** *(2006.01)* ***A61P 35/00*** *(2006.01)*

(86) International application number:
**PCT/CN2018/115235**

(87) International publication number:
**WO 2019/096127 (23.05.2019 Gazette 2019/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.11.2017 CN 201711124057**

(71) Applicant: **Guangzhou Institute Of Biomedicine And Health, Chinese Academy Of Sciences Guangzhou, Guangdong 510530 (CN)**

(72) Inventors:
- **LI, Yi Guangzhou Guangdong 510530 (CN)**
- **ZHOU, Peipei Guangzhou Guangdong 510530 (CN)**

(74) Representative: **Mewburn Ellis LLP Aurora Building Counterslip Bristol BS1 6BX (GB)**

# (54) GENETICALLY ENGINEERED GAMMA DELTA T CELL

(57) Provided is a genetically engineered γδT cell, which is characterized in that a high-affinity αβTCR gene is transferred into the γδT cell, and the affinity of the high-affinity αβTCR to the specific pMHC thereof is at least two times of that of a wild-type αβTCR corresponding thereto. Further provided are a use of and a preparation method for the γδT cell.



Printed by Jouve, 75001 PARIS (FR)

## Description

### Technical field

The present invention relates to the field of biotechnology, and in particular, to engineered γδT cells, and uses and preparation methods for the cells.

### Background

Malignant tumors are one of the most dangerous diseases that endanger human health. Every year, about 8 million people die from cancer worldwide, and 6 people are diagnosed as having malignant tumors per 6 minutes in China. The development of new drugs and treatments for malignant tumors has become the global focus. Cancer immunotherapy was rated as the most important scientific breakthrough in 2013 by "Science", among which adoptive cell immunotherapy is particularly interesting.

For adoptive cellular immunotherapy, the patient's own immune cells are expanded or modified *in vitro,* and then introduced back into the body, so as to achieve the purpose of treating tumors. This therapy has advantages, such as high specificity and few side effects, and is especially suitable for patients who cannot receive other treatments at the late stage. At present, T lymphocytes have become a new star of adoptive cellular immunotherapy.

T lymphocytes are divided into two major categories of αβT and γδT cells based on the structure of T cell receptor (TCR) on their surface. Human γδT cells are a unique subset of T lymphocytes that are conservatively evolved, their TCR receptors are composed of γ chains and δ chains; and they have anti-infection and anti-tumor effects in the immune monitoring of the body. There are many types and a variety of subtypes for γδT cells. Among them, the Vδ chain has 1-8 different subtypes, and the Vγ chain has 1-9 different subtypes. In human peripheral blood, γδT cells account for 1%-5% of the entire T lymphocytes, in which Vγ9Vδ2γδT cells account for 50%-95% of the entire γδT cells. Compared with αβT cells, γδT cells recognize more types of antigens, and are not restricted by major histocompatibility complex (MHC).

At present, the research on T cell adoptive immunotherapy mainly focuses on CAT-T therapy and TCR-T therapy. Among them, engineered TCR gene-modify T cells are mainly used in TCR-T therapy. Compared with the currently used αβTCR-transduced αβT cell protocol, αβTCR transduced γδT cells will naturaly possess advantages: its exogenous αβTCR hardly mismatches the endogenous γ chain and δ chain of γδT cells, thereby avoiding likely caused autoimmune reaction to the body.

In 2009, A Hiasa et al. [A Hiasa1 Gene Therapy (2009) 16, 620-628] transduced αβTCR to γδT cells for the first time, and found that αβTCR-transduced γδT cells can transmit activation signals to cells through endogenous yδTCR, but also can respond to specific antigens through exogenous αβTCR At the same time, from activation dynamics, αβTCR-transduced γδT cells respond quickly, compared with αβTCR-transduced αβT cells. However, unmodified wild-type αβTCR is used in Hiasa's solution. If only αβTCR is transduced into γδT cells, specific recognition of tumor antigens can not be achieved. Eventually, CD8 molecules have to be transduced to assist recognition, which undoubtedly increases the instability of the cell, and additional gene editing will bring more unpredictability and potential risk.

Therefore, there is an urgent need in the art for γδ T cells that specifically recognize tumor antigens without co-transforming other CD molecules.

### Summary of the Invention

The object of the present invention is to provide a γδT cell that can specifically recognize a tumor antigen without co-transforming other CD molecules.

The object of the present invention is also to provide a preparation method and use of the γδT cells.

In the first aspect, the present invention provides a genetically modified γδT cell, into which a gene of high-affinity αβTCR has been transduced, and the affinity of the high-affinity αβTCR to its specific pMHC is at least two times that of the corresponding wild-type αβTCR.

In a preferred embodiment, the genetically modified γδT cells can specifically recognize a tumor antigen without exogenous CD8 molecules; preferably, the genetically modified γδT cells can specifically recognize a tumor antigen without any exogenous CD molecules; and most preferably, the genetically modified γδT cells can specifically recognize a tumor antigen without any exogenous auxiliary molecules.

In a specific embodiment, the equilibrium dissociation constant value KD of the affinity of the high-affinity αβ TCR to its specific pMHC is ≤ 10 μM.

In a specific embodiment, the equilibrium dissociation constant value KD of the affinity of the high-affinity αβTCR to its specific pMHC is ≤ 4 μM; for example, 5 nM ≤ KD ≤ 4000 nM, 50 nM ≤ KD ≤ 4000 nM, 100 nM ≤ KD ≤ 4000 nM, 400 nM ≤ KD ≤ 4000 nM, 450 nM ≤ KD ≤ 4000 nM, 400 nM ≤ KD ≤ 3000 nM, 500 nM ≤ KD ≤ 3000 nM, 1000 nM ≤ KD ≤ 3000 nM or 1500 nM ≤ KD ≤ 3000 nM.

In a specific embodiment, the equilibrium dissociation constant value KD of the affinity of the high affinity αβ TCR to its specific pMHC is: 1 nM ≤ KD ≤ 2000 nM; for example, 100 nM ≤ KD ≤ 2000 nM, 400 nM ≤ KD ≤ 2000 nM, 450 nM ≤ KD ≤ 2000 nM, 500 nM ≤ KD ≤ 2000 nM, 1000 nM ≤ KD ≤ 2000 nM or 1500 nM ≤ KD ≤ 2000 nM.

In a preferred embodiment, the gene of the high affinity αβ TCR is of full-length.

In a preferred embodiment, the nucleic acid sequence of the α-chain of the high-affinity αβTCR is SEQ ID NO: 5, and the nucleic acid sequence of the β-chain is SEQ ID NO: 7; or, the nucleic acid sequence of the α-chain is SEQ ID NO: 9, and the nucleic acid sequence of the β chain is SEQ ID NO: 11; or, the nucleic acid sequence of the α chain is SEQ ID NO: 13, and the nucleic acid sequence of the β chain is SEQ ID NO: 15; or, the nucleic acid sequence of the α chain is SEQ ID: NO: 17, and the nucleic acid sequence of the β chain is SEQ ID NO: 19; or, the nucleic acid sequence of the α chain is SEQ ID NO: 21, and the nucleic acid sequence of the β chain is SEQ ID NO: 23.

In a preferred embodiment, the high affinity αβ TCR is transduced into the yδ T cells by viral vectors, including but not limited to lentiviral vectors.

In a preferred embodiment, the yδ pair of the γδT cell is Vγ1-9 and Vδ1-8.

In a preferred embodiment, the cells are Vγ9Vδ2γδT cells.

In a specific embodiment, the γδT cells are used to prepare medicaments for treating a tumor or an infectious disease.

In a specific embodiment, the infection is a viral infection or a bacterial infection.

In a preferred embodiment, the γδ T cells are derived from a treated subject itself or from a healthy donor.

In a second aspect, the present invention provides uses of the γδT cell of the first aspect for preparing a medicament for treating a tumor or infectious disease.

In a preferred embodiment, the infectious disease is a viral infection or a bacterial infection.

In a third aspect, the present invention provides a pharmaceutical composition, wherein the pharmaceutical composition comprises a pharmaceutically acceptable carrier and the yδ T cell of the first aspect.

In a fourth aspect, the present invention provides a method for treating a disease, comprising administering to a subject in need thereof an appropriate amount of the yδ T cell of the first aspect or the pharmaceutical composition of the third aspect.

In a preferred embodiment, the disease is a tumor or an infectious disease.

In a preferred embodiment, the infectious disease is a viral infection or a bacterial infection.

In a fifth aspect, the present invention provides a method for preparing the γδT cell of the first aspect, the method comprising the following steps:

(i) transducing a gene of high-affinity αβTCR into a γδT cell;
(ii) culturing the γδT cell described in (i).

In a preferred embodiment, the affinity of the high-affinity αβTCR to its specific pMHC is at least two times that of the corresponding wild-type αβTCR.

It should be understood that, within the scope of the present invention, the above technical features of the present invention and the technical features specifically described in the following (e.g., examples) can be combined with each other, thereby forming a new or preferred technical solution, which is not necessary to be repeated one by one.

## Description of Figures

Figures 1a-x show the nucleic acid sequence of α chain and corresponding amino acid sequence and the nucleic acid sequence of of β chain and corresponding amino acid sequence of 32μMTCR (wild type), 4μMTCR, 1.07μMTCR, 450nMTCR, 84nMTCR and 5nMTCR against 1G4, respectively;

Figure 2 shows the cytokine (IFN-y) release responses of groups of yδT cells transduced with 1G4TCR of different affinities on T2 cells loaded with a specific short peptide, a control short peptide and no short peptide;

Figure 3 shows the cytokine (IFN-y) release responses of groups of yδT cells transduced with 1G4TCR of different affinities on tumor cell lines of 1G4TCR-transduced γδT cell groups with different affinities;

Figure 4 shows the specific killing comparison of groups of the 1G4TCR-transduced γδT cells on tumor cell lines: LDH detection;

Figure 5 shows the difference in killing effects of 1G4TCR-transduced γδT cells on tumor cell lines by detected by luciferase reaction;

Figure 6 shows the killing kinetic responses of yδT cells transduced with 1G4TCR of different affinities on tumor cell lines detected in real time.

## Modes for carrying out the invention

After extensive and in-depth research, the inventors unexpectedly discovered that γδT cells transduced with high-affinity αβTCR can specifically recognize tumor antigens without co-transforming other CD molecules. And such modified γδT cells showed unexpected and more excellent effects in experiment of activation function and killing function. Such modified cells are obviously more advantageous in terms of stability, since it is not necessary to co-transform other CD molecules, thereby greatly reducing the unpredictability and potential risks in the preparation process. On this basis, the present invention has been completed.

## Terms

pMHC: major histocompatibility complex, i.e., MHC, is a generic name for all biocompatibility complex antigens and is a molecule encoded by the MHC gene family. Human MHC is often called HLA gene or HLA complex. A tumor antigen will be proteolytically processed into polypeptide fragments of 8-16 amino acid in length, i.e., CTL epitopes, in the cells. The polypeptide fragments combine with MHC molecules in the endoplasmic reticulum cavity to form a peptide-MHC complex, i.e., pMHC. pMHC is finally presented on the cell surface for recognition by the αβT cell receptor (αβTCR) on the surface of CD8 + T cells. Different αβTCR can recognize different pMHC molecules, and αβTCR is specific for pMHC recognition. Wild-type αβTCR exhibits a certain affinity for its specific pMHC.
High affinity αβTCR: refers to a αβTCR, whose affinity to its specific pMHC molecule is at least twice that of the corresponding wild-type αβTCR to the aforementioned pMHC molecule.
Tumor: refers to all types of cancer cell growth or carcinogenic processes, metastatic tissue or malignant transformed cells, tissues or organs, regardless of pathological type or stage of infection. Examples of tumors include, without limitation, solid tumors, soft tissue tumors, and metastatic lesions. Examples of solid tumors include: malignant tumors of different organs or systems, such as sarcoma, lung squamous cell carcinoma and cancer. For example: infected prostate, lung, breast, lymph, gastrointestinal (e.g., colon), and genitourinary tract (e.g., kidney, epithelial cells), pharynx. Lung squamous cell carcinoma includes malignant tumors, for example, most colon cancers, rectal cancer, renal cell cancer, liver cancer, non-small cell cancer of the lung, small intestine cancer, and esophageal cancer. Based on the teachings of the present invention, a skilled person in the art can understand that the high-affinity αβTCR-transduced γδT cells of the present invention can specifically recognize a tumor antigen without or without expressing exogenous auxiliary molecules, such as CD8 molecules, thereby achieving killing effects on tumor cells. Therefore, the various genetically modified γδT cells prepared by employing the idea of the present invention have potential therapeutic effects on various tumors.
Pharmaceutical carrier: also named as an excipient or stabilizer, the dosage and concentration of which are not toxic to the cells or individuals exposed to it. The physiologically acceptable carrier is generally a pH buffered aqueous solution. Examples of a physiologically acceptable carrier include buffers such as phosphates, citrates, and other organic acids; antioxidants, including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides and other sugars, including glucose, mannose or dextrin; complexing agents such as EDTA; sugar alcohols such as mannitol or sorbitol; salt-forming counterions such as sodium; and / or nonionic surfactants such as TWEEN™, polyethylene glycol (PEG) and PLURONICS™.

## Genetically modified γδT cells

After extensive and in-depth research, the present inventors obtained a genetically modified γδT cell with excellent performance, into which a gene of high affinity αβTCR is transduced, and the affinity of the high affinity αβTCR to its specific pMHC is at least twice that of the corresponding wild-type αβTCR. Therefore, the yδ T cells of the present invention can specifically recognize a tumor antigen without or without expressing exogenous CD8 molecules; preferably, the yδ T cells of the present invention can specifically recognize a tumor antigen without or without expressing any exogenous CD molecules (e.g., but not limited to CD8, CD4 molecules); and most preferably, the γδT cells of the present invention can specifically recognize a tumor antigen without or without expressing any exogenous auxiliary molecules.
In particular, the equilibrium dissociation constant value KD of affinity of the high-affinity αβTCR to its specific pMHC ≤ 10 μM. More particularly, the equilibrium dissociation constant value KD of the affinity of the high-affinity αβTCR to its specific pMHC is ≤ 4 μM; for example, 5 nM ≤ KD ≤ 4000 nM, 50 nM ≤ KD ≤ 4000 nM, 100 nM ≤ KD ≤ 4000 nM, 400nM ≤ KD ≤ 4000nM, 450 nM ≤ KD ≤ 4000 nM, 400 nM ≤ KD ≤ 3000 nM, 500 nM ≤ KD ≤ 3000 nM, 1000 nM ≤ KD ≤ 3000 nM or 1500 nM ≤ KD ≤ 3000 nM.
In another preferred example, the equilibrium dissociation constant value KD of the affinity of the high affinity αβ TCR to its specific pMHC is: 1 nM ≤ KD ≤ 2000 nM; for example, 100 nM ≤ KD ≤ 2000 nM, 400 nM ≤ KD ≤ 2000 nM, 450 nM ≤ KD ≤ 2000 nM, 500 nM ≤ KD ≤ 2000 nM, 1000 nM ≤ KD ≤ 2000 nM or 1500 nM ≤ KD ≤ 2000 nM.

Manners for determining the affinity between αβ TCR and its specific pMHC are well-known to a skilled person in the art. The binding affinity can be determined by any suitable method and using any suitable instrument, including but not limited to Biacore SPR or ForteBio, etc. For example, the binding affinity can be determined according to the instructions of the relevant instrument. It should be noted that when determining the binding affinity, both of αβTCR and pMHC are in a form of soluble protein, that is, both of αβTCR and pMHC are soluble in water. Methods for obtaining αβTCR and pMHC complexes in a soluble form are well-known to a skilled person, such as literature (Jonathan M. Boulter et al, 2003, Protein Engineering, 16 (9): 707-711) or patent literature PCT/CN2016/077680.

It is well known that the affinity is inversely proportional to the equilibrium dissociation constant KD. Therefore, if the affinity of TCR is doubled, the KD value will be reduced by 50%. The conversion relationship between KD value units are well-known to a skilled person in the art, that is, 1 M = 1000 μM, 1 μM = 1000 nM, and 1 nM = 1000 pM. The binding constant is expressed as ka, and the dissociation constant is expressed as kd, KD = kd / ka. T1 / 2 (half-life) is calculated as In2 divided by the dissociation constant (kd). Therefore, if T1/2 is doubled, kd will be reduced by 50%. The same test protocol is advantageously employed to detect the binding affinity or binding half-life of a given TCR for several times, for example 3 or more times, and the average of the results are taken.

In a preferred embodiment, such detection is performed using Biacore SPR method. Biacore SPR: Surface plasmon resonance measurement method, that is, the resonance principle that the surface plasmon is sensitive to changes in the refractive index of a biosensor at 150 nm is empolyed to respond to changes in the concentration of the molecule on the surface of a biosensor in a real-time, non-invasive manner, in a real-time, non-invasive way, thereby detecting the interaction between the ligand and the analyte on the biosensor chip.

T lymphocytes are divided into two major categories of αβT and γδT cells based on the structure of T cell receptor (TCR) on their surface. The TCR on the surface of αβT cells is composed of α chain and β chain, which is called as αβTCR; the TCR on the surface of yδT cell is composed of γ chain and δ chain, which is called as γδTCR. Among them, the Vδ chain has 1-8 different subtypes, and the Vγ chain has 1-9 different subtypes. Among them, Vγ9Vδ2γδT cells are also called as Vδ2T cells, which can be used interchangeably in the present invention. In a preferred embodiment of the present invention, the gene of a high-affinity αβ TCR transduced into a yδ T cell is of full-length, including an extracellular region, transmembrane region and cytoplasmic region.

Preferably, the high affinity αβ TCR is transduced into a yδ T cell by viral vectors. Viral vectors include but are not limited to adenovirus vectors, adeno-associated virus (AAV) vectors, herpes virus vectors, retrovirus vectors, lentivirus vectors, and baculovirus vectors. In a preferred embodiment of the present invention, the used vector is a lentiviral vector. Preferably, the gene of a high affinity αβ TCR is integrated into the chromosome of a yδ T cell.

The γδT cells of the present invention can be used to treat a tumor or infectious disease. The tumor includes but not limited to liver cancer, lung cancer, gastric cancer, malignant melanoma, prostate cancer, bladder cancer, breast cancer, multiple myeloma, hepatocellular carcinoma, oral squamous cell carcinoma and esophageal cancer, and the like. The infections include viral infections, bacterial infections, fungal infections and protozoan infections. The γδT cells of the present invention can be applied to adoptive immunotherapy, and many suitable methods for adoptive therapy are known to a skilled person in the art (e.g., Rosenberg et al., (2008) Nat Rev Cancer 8 (4): 299-308).

The recognition of an antigen by a γδT cell is not limited by the complex formed by major histocompatibility complex (MHC) and specific antigen short peptides, the γδT cell has extensive anti-tumor and anti-infective properties, and after being stimulated by the antigen, it can respond quickly and expand in great quantity, so as to meet the clinical dosage. Therefore, when treating the above-mentioned diseases, the γδT cells can be extracted not only from the patient but also from other healthy donors. The T cells may be derived from T cells isolated from a subject, or may be a part of a mixed cell population isolated from the subject, such as a part of a peripheral blood lymphocyte (PBL) population. For example, the cells can be isolated from peripheral blood mononuclear cells (PBMC). Alternatively, the cells of the invention may also be or derived from stem cells, such as hematopoietic stem cells (HSC).

A patient can be treated by isolating yδ T cells from the patient with a relevent disease, introducing high-affinity αβ TCR into the T cells, and then reintroducing these genetically modified cells into the patient. It is also possible to isolate γδT cells from a healthy people, and introduce high-affinity αβTCR into the γδT cells, so as to obtain modified γδT cells for future use and reintroduce to a people in need thereof. The amount of reinfusion can be determined by a physician.

Therefore, the yδ T cells of the present invention can be obtained from various sources, including but not limited to commercial sources.

It should be understood that an internationally accepted single letter is used herein to represent the name of an amino acid, and the corresponding three-letters abbreviation of amino acids are: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), Val (V);

**Advantages of the invention:**

1. The γδT cells of the present invention, into whcih a high-affinity αβTCR is transduced can specifically recognize a tumor antigen without being co-transformed with other CD molecules, and the preparation method and process thereof are relatively convenient.
2. The γδT cells of the present invention, into whcih a high-affinity αβTCR is transduced exhibit unexpected and excellent activation functions and killing effects on target cells.

The following specific examples further illustrate the present invention. It should be understood that these examples are only used to illustrate the present invention and not to limit the scope of the present invention. Experimental methods without specific conditions in the following examples are generally performed in accordance with conventional conditions, for example (Sambrook and Russell et al. Molecular cloning: Molecular Cloning-A Laboratory Manual (Third Edition) (2001) CSHL The conditions described in the company) or the conditions recommended by the manufacturer. Unless otherwise stated, percentages and parts are calculated by weight. Unless otherwise specified, the experimental materials and reagents used in the following examples can be obtained from commercially available channels.

Before describing the present invention, it should be understood that the present invention is not limited to the described specific methods and experimental conditions, since such methods and conditions may vary. It should also be understood that the terminology used herein is for the purpose of describing specific embodiments and is not intended to be limiting, and the scope of the present invention will be limited only by the appended claims.

Unless otherwise defined, all technical and scientific terms used herein have the same meaning as commonly understood by a skilled person in the art to which this invention belongs.

Although any methods and materials similar or equivalent to those described in the present invention can be used in practicing or testing of the present invention, the preferred methods and materials are exemplified herein.

**Example 1. Lentiviral packaging of T cell receptor gene**

The full-length genes of α and β chains of a TCR were connected to a lentivirus expression vector.

**Sources of main materials**

Three lentivirus packaging plasmids pMDLg / pRRE (Cat. No. 12251), pRSV-REV (Cat. No. 12253), pMD2.G (Cat. No. 12259) were purchased from addgene; and the plasmid PEI (Cat. No. 23966) was purchased from polyscience.

Liposome transfection was used by the inventors to co-transfect the 293T packaging cells with the third-generation of lentiviral system 4 plasmid (3 plasmids using pLenti (addgene) as the lentiviral vector, and containing other components necessary for constructing infectious but non-replicating lentiviral particles) to prepare lentivirus containing the target T cell receptor gene.

**Methods**

Cell culture: In a 15 cm dish, a suspension of 1.5-2 * $10^7$ 293T cells (DMEM medium containing 10% FBS) was evenly plated, placed at 37°C, under 5% $CO_2$ overnight; and when about 80% confluence was achieved, lentivirus packaging was conducted.

Lentivirus packaging: the expression plasmid PLenti-TCRα-P2A-TCRβ (15 ug/dish) and packaging plasmids pMDLg/pRRE (5 ug/dish), pRSV-REV (5 ug/dish) and pMD2.G (5 ug/dish) and 1800 microliters of opti-MEM medium was mixed thoroughly, and placed at room temperature for 5 minutes to form a DNA mixture; PEI (60 ug/dish) and 1800 microliters of opti-MEM medium were taken and mixed thoroughly, and placed at room temperature for 5 minutes to form a PEI mixture solution. The DNA mixture and the PEI mixture were mixed together, placed for 30 minutes at room temperature, then 3150 microliters of opti-MEM medium was added, mixed well and added to a 293T cell culture dish, in which DMEM medium had been changed to 11.25 ml of opti-MEM medium, mixed gently and incubated for 7-8 hours at 37°C under 5% $CO_2$, and then the transfection medium was changed to DMEM medium containing 10% FBS. The supernatant virus liquid was collected at 48 hours and 72 hours after transfection, respectively. The collected virus liquid was centrifuged at 3000g for 10-15 minutes to remove cell debris; after Alteration through a 0.45 micron filter, a concentration tube with 50KD cut-off value was used to conduct centrifugation at low-temperature. The virus liquid obtained from 1 dish was finally concentrated to 1 ml of virus concentrate, and then divided and stored at -80°C.

**Example 2. Transduction of yδT cells with target gene**

γδT cells were transduced with the lentiviral expression vector containing the gene of interest constructed in Example 1.

Sources of main reagents and material

Human peripheral blood mononuclear cells (PBMC) were from healthy donors. Zoledronete was purchased from NOVARTIS, batch number: S0425, imported drug registration account: H20140218; Recombinant Human Interleukin-2 (IL-2) for Injection: Shandong Quangang Pharmaceutical Co., Ltd., batch number: 201407004; IL-7: PEPROTECH, Cat. No.: AF-200-07; IL-15: PEPROTECH, Cat. No.: AF-200-15; IL-21: PEPROTECH, Cat. No.: AF- 200-21; medium: 10% FBS (Australian fetal bovine serum, inactivated): purchased from Gibco, Cat. No. 1828728; PRMI1640 (Gibco, Life Technologies) purchased from Gibco, Cat. No. 8117107; flow cytometer: BD FACSAriaIII.

Methods

(a) Vγ9Vδ2γδT cell stimulation: PBMCs from a healthy donor were resuspended in a complete medium, RPMI1640 + 10% Au + 1% PS and mixed homogeneously. The cells were divided into a 24-well plate at a concentration of 2 * $10^6$ / ml. The initial stimulation is recorded as D0, and zoledronete (working concentration was 5 uM) is added to the PBMCs in each wells, and the cytokine IL-2 (working concentration was 100 IU/ml) was also supplemented; and in such system, directed induction and activation of Vγ9Vδ2γδT cells present in PBMC was initiated. The plate was placed in a 37°C, 5% CO2 incubator. In the follow-up culture, half of the medium was exchanged by adding fresh medium and IL-2 every 2-3 days.

(b) Lentiviral transduction of Vγ9Vδ2γδT cells: At D2/D3 after activation, the concentrated lentivirus with the target gene was added into a 200 μl of infection system in a 48-well plate at a proportion of MOI = 5. 10 ug/ml of protamine was also supplemented at the same time. At D4, Vγ9Vδ2γδT cells transduced with lentivirus were transferred from a 200-microliter system in the 48-well plate to 1-ml system in a 24-well plate for cultivation.

**Example 3. Determination of affinity between αβTCR and its specific pMHC**

The binding affinity can be determined by any suitable method using any suitable instrument, including but not limited to Biacore SPR or ForteBio, etc. For example, it can be determined according to the instruction manual of the relevant instrument. It should be noted that both αβTCR and pMHC can exist in a form of soluble protein when determining binding affinity. Methods for obtaining αβTCR and pMHC complexes in a soluble form are well-know to a skilled person in the art, such as described in a literature (Jonathan M. Bouter et al, 2003, Protein Engineering, 16 (9): 707-711) or patent literature PCT/CN2016/077680.

The soluble pMHC complex can be prepared according to the following method: MHC heavy and light chains are expressed by using inclusion bodies, then short peptides are synthesized, and the dissolved MHC heavy chain and light chain as well as short peptides were added into a renaturation buffer and purified. The biotinylated pMHC molecules can be aliquoted and stored at -80°C.

In this example, the affinity between the αβTCR protein and its specific pMHC was determined by using a Biacore instrument from GE. The streptavidin was fixed to the surface of a CM5 chip by amino-coupling method, and the fixing amount was greater than several hundred RU. After the biotinylated non-specific pMHC and specific pMHC were captured in the reference channel and the detection channel, respectively, biotin was used to block remaining biotin binding sites of streptavidin. The TCR was diluted into several suitable concentrations in a running buffer and injected sequentially. The dissociation time will depend on the TCR affinity. The analysis software of Biacore was usd to analyze the measured data, the 1: 1 combining model was used to fit the obtained kinetic parameters, and the equilibrium dissociation constant KD = kd / ka.

**Example 4. Activation experiment of specific response of high affinity 1G4TCR transduced γδT cells to T2 cells**

This experiment was conducted to verify the activation of γδ T cells transduced with high affinity 1G4 TCR that specifically respond to target cells T2. The effects were significantly better than that of yδT cells transduced with wild-type 1G4TCR. The IFN-γ production was detected by ELISPOT test as the readout value of TCR-transduced γδT cell activation.

Sources of main reagents and material

10% FBS (Gibco, Catalog number 16000-044), RPMI 1640 (Gibco, Catalog number C11875500bt), PVDF ELISPOT 96-well plate (Merck Millipore, Catalog number MSIPS4510), human IFN-γ ELISPOT PVDF-enzyme kit (BD) containing all other required reagents (capture and detection antibody, streptavidin-alkaline phosphatase and BCIP / NBT solution), Elispot 's Kit (BD™ ELISPOT ELISPOT Set Instruction Manual Catalog number: 551282), short peptide: P1B specific for the antigen NY-ESO-1 157-165, sequence of which is SLLMWITQC, HLA genotype of which is A0201, P72A for the control antigen GP100 , sequence of which is YLEPGPVTA, and corresponding HLA genotype of which is A0201.

**Methods**

**Preparation of target cells**

The target cell in this example is a lymphoblastoma T2 cell. The HLA type of the cell is A0201, but it does not express the antigen NY-ESO-1 specific for 1G4TCR; T2 cells loaded with antigen NY-ESO-1 short peptide P1B (loading concentration was 0.1μM \ 0.01μM \ 0.0001μM) and control peptide P72A (loading concentration was 0.1 μM), and T2 cells without short peptides were used as the three types of target cells in this experiment.

**Preparation of effector cells**

Different groups of effector cells were prepared: Vδ2T cells without being transduced with 1G4TCR and Vδ2T cells transduced with 1G4TCR of different affinities. The type of yδT cells is Vγ9Vδ2γδT cells (Vδ2T cells). The 1G4TCR (Yangbing Zhao, * The Journal of Immunology, 2007, 179: 5845-5854) is a high-affinity αβ TCR, and the affinity of the TCR used in this example is 32 μM (wild type) (the nucleic acid sequence of α chain is SEQ ID NO: 1, the amino acid sequence of α chain is SEQ ID NO: 2; and the nucleic acid sequence of β chain is SEQ ID NO: 3, the amino acid sequence of β chain is SEQ ID NO: 4), 4 μM (the nucleic acid sequence of α chain is SEQ ID NO : 5, the amino acid sequence of α chain is SEQ ID NO: 6; and the nucleic acid sequence of β chain is SEQ ID NO: 7, the amino acid sequence of β chain is SEQ ID NO: 8), 1.07 μM (the nucleic acid sequence of α chain is SEQ ID NO: 9, the amino acid sequence of α chain is SEQ ID NO: 10; the nucleic acid sequence of β chain is SEQ ID NO: 11, the amino acid sequence of β chain is SEQ ID NO: 12), 450nM (the nucleic acid sequence of α chain is SEQ ID NO: 13, the amino acid sequence of α chain SEQ ID NO: 14; and the nucleic acid sequence of of β chain is SEQ ID NO: 15, the amino acid sequence of β chain is SEQ ID NO: 16), 84nM (the nucleic acid sequence of α chain is SEQ ID NO: 17, the amino acid sequence of α chain is SEQ ID NO: 18; the nucleic acid sequence of β chain is SEQ ID NO: 19, the amino acid sequence of β chain is SEQ ID NO: 20) and 5nM (the nucleic acid sequence of α chain is SEQ ID NO: 21, the amino acid sequence of α chain is SEQ ID NO: 22; and the nucleic acid sequence of β chain is SEQ ID NO: 23, and the amino acid sequence of β chain is SEQ ID NO: 24).

**ELISPOT operation**

Plates were repared according to the instruction provided by the manufacturer as follows: anti-human IFN-γ capture antibody was diluted at 1: 200 by using 10 ml of sterile PBS per plate, and then 100 μl of diluted capture antibody was added to each well. The plate was incubated at 4°C overnight. After incubation, the plate was washed to remove excess capture antibody. 100 microliters of RPMI1640 medium containing 10% FBS was added to each well and the plate was incubated at room temperature for 2 hours to block the plate. The medium was then washed away from the plate, and any residual washing buffer was removed from the ELISPOT plate using paper. The effector cells and target cells prepared above were added to the ELISPOT well plate through centrifugation and counting: for 100 μl of target cells, 2 * $10^5$ cells per ml (20,000 cells per well), and for 100 μl of effector cells, 2 * $10^4$ cells per ml (2000 cells per well). Note: The short peptide dilution produced by the gradient dilution was directly used to resuspend the T2 cells. In all wells, additions in triplicate were conducted.

Then the plate was incubated at 4°C overnight (37°C, 5% $CO_2$). On the next day, the culture medium was discarded, the plate was washed twice with double-distilled water, and then washed with the washing buffer for three times. The residual washing buffer was removed on a paper tissue. Then the primary antibody was diluted with PBS containing 10% FBS and added to each well at 100 μl per well. The plate was incubated at room temperature for 2 hours, the washed with a washing buffer for three times, and the excess washing buffer was removed on a paper tissue.

Streptavidin-alkaline phosphatase was diluted at 1: 100 with PBS containing 10% FBS, 100 μl of diluted streptavidin-alkaline phosphatase was added to each well, and the plate was incubated at room temperature for 1 hour. Then the plate was washed for 3 times with a washing buffer and 2 times with PBS, and pat the excess washing buffer and PBS were removed on a paper tissue. After washing, 100 μl / well of BCIP / NBT solution provided by the kit was added for development. The plate was covered with a tin foil during development to avoid light, and incubated for 5-15 minutes. During this period, spots on the developed plate were routinely checked to determine the best time to quench the reaction. The BCIP / NBT solution was removed and the plate was rinsed with double distilled water to stop quench the development reaction, and spin-dried. And then the bottom of the plate was removed. The plate was dried at room temperature until each well is completely dry, and then the immunospot plate counter (CTL, Cellular Technology Limited) was used to count the number of spots formed on the bottom membrane in the plate.

**Results**

The release of IFN-γ from the yδ T cells transduced with the TCR of the present invention in response to T2 cells loaded with NY-ESO-1 157-165 P1B SLLMWITQC short peptide and T2 cells loaded with the control peptide P72A was detected by ELISPOT experiment (as described above). The number of detected spots was analyzed and plotted using the software GraphPad Prism7.

The experimental results are shown in Figure 2, in which the X-axis indicates the effector cell group with different treatments, and the Y-axis indicates the number of spots producing IFN-γ. The greater the number of spots, the greater the activation effects of the corresponding effector group when incubated with the target cells. γδT cells transduced with high-affinity αβTCR exhibited strong specific activation effects against T2 cells loaded with specific short peptides, which are significantly higher than those of yδT cells transduced with wild-type αβTCR, thereby releasing more IFN- γ. For example, when the concentration of the short peptide is 0.1 μM, for γδT cells transduced with αβTCR with an affinity KD of 4 μM-5 nM, the number of spots of activating IFN-γ was 400-600, most of which were nearly 600, however, for γδT cells transduced with wild-type αβTCR, the number of spots of activating IFN-γ was only about 150. It shows that when the affinity value of αβTCR is 4 μM ≤ KD ≤ 5 nM, the transduced γδT cells can show a strong activation response against the target cells, while there is basically no response to T2 cells loaded with non-specific short peptides and empty T2 cells. Vδ2T cells that have not been transduced with exogenous αβTCR exhibited basically no response to related tumor cell lines.

**Example 5. Activation experiment of high affinity 1G4TCR-transduced γδT cells to specifically respond to tumor cell lines**

This experiment was conducted to verify the activation of high affinity 1G4 TCR-transduced γδT cells that specifically respond to tumor cell lines. The achieved effects were significantly better than thoes of yδT cells transduced with wild-type 1G4TCR. The IFN-γ production detected by the ELISPOT test was used as the readout value of TCR-transduced γδT cell activation. The specific operation of ELISPOT experiment is as described in Example 4.

**Sources of main reagents and material**

10% FBS (Gibco, Catalog number 16000-044), RPMI 1640 (Gibco, Catalog number C11875500bt), PVDF ELISPOT 96-well plate (Merck Millipore, Catalog number MSIPS4510), human IFN-γ ELISPOT PVDF-enzyme kit (BD) containing all other required reagents (capture and detection antibody, streptavidin-alkaline phosphatase and BCIP / NBT solution), Elispot 's Kit (BD™ ELISPOT ELISPOT Set Instruction Manual Catalog number: 551282), tumor cell line: IM9 is multiple myeloma, HLA genotype of which is A0201, also express NY-ESO-1 antigen; U266B1 is myeloma, plasma cells, HLA genotype of which is A0201, also expresses NY-ESO-1 antigen; NCI-H1299 is a lung cancer cell line that expresses NY-ESO-1 antigen, but HLA genotype of which is not A0201, as a control; LCLs are immortalized lymphoblastoid cell lines, which does not express NY-ESO-1 antigen, and the HLA genotype of which is not A0201, as a control. The above tumor cell lines were purchased from ATCC.

**Methods**

**Preparation of target cells**

The target cells of this experimental example were IM9 and U266B1 as the positive tumor cell line of this experiment. The HLA genotype of these two cell lines is A0201, and they also express the antigen NY-ESO-1; NCI-H1299 expresses NY-ESO -1 antigen, but the HLA genotype is not A0201; LCLs neither express NY-ESO-1 antigen, nor the HLA genotype is A0201, these two cells were used as negative tumor cell lines in this experiment.

**Preparation of effector cells**

Preparation of effector cells was described in the examples. Different groups of effector cells were prepared: Vδ2T cells without being transduced with 1G4TCR and Vδ2T cells transduced with 1G4TCRs of different affinities of 32μM (wild type), 4μM, 1.07μM, 450nM, 84nM and 5nM.

The experimental results are shown in Figure 3. γδT cells transduced with high affinity αβTCR exhibited strong activation effects against positive tumor cell lines, and the effects were significantly higher than those of γδT cells transduced with wild-type αβTCR, thereby releasing more IFN-γ . For example, for the positive cell line IM9, γδT cells transduced with wild-type αβTCR basically exhibited no specific activation response. When the high-affinity αβTCR with a KD value of 4 μM was transduced, the specific activation response was enhanced, and the number of IFN-γ spots is about 100. With the increase of affinity, the activation response was obviously enhanced. When the KD value was 5 nM, the number of IFN-γ spots reached about 650. While there was basically no response to the negative tumor cell line, indicating that a very good specificity. Vδ2T cells that were not transduced with exogenous αβTCR basically did not respond to the relevant tumor cell lines.

**Example 6. Non-radioactive cytotoxicity experiment**

Different from the traditional 51Cr release method, this experiment uses a colorimetric-based detection method-CytoTox 96® non-radioactive cytotoxicity test to quantitatively measure the lactate dehydrogenase (LDH) released during cell lysis. The reaction principle is: cells will release LDH into the supernatant of the medium due to lysis, which can be detected by a 30-minute coupled enzyme reaction. In the enzyme reaction, LDH can convert a tetrazolium salt (INT) into red formazan. The amount of red product produced is directly proportional to the number of lysed cells. A standard 96-well plate reader was used to collect the visible light absorbance data at 490 nm. This method is used to reflect the lethality to target cells when effector cells and target cells are incubated together.

**Materials**

CytoTox 96® non-radioactive cytotoxicity test (Promega; Catalog No .: G1780) kit and supporting substrate mixture, test buffer, lysis solution and stop buffer. Medium: 10% FBS (Gibco, Catalog number: 16000-044), added to RPMI1640 (Gibco, Catalog number: C11875500bt) medium without phenol red. Note: FBS has been inactivated before use. Round bottom 96-well tissue culture plate (Corning, Catalog number: 3799); 96-well immunoplate (Thermo, Catalog number: 167008). Tumor cell lines: A375 melanoma (HLA type of which is A0201, and which expresses antigen NY-ESO-1) and NCI-H1650 lung cancer cell line (HLA type of which is A0201 and which does not express antigen NY-ESO-1) were purchased from ATCC; NCI -H1299 lung cancer cells (HLA type of which is A0201 and which expresses antigen NY-ESO-1), which is in-house modified from the wild type purchased from the ATCC.

**Methods**

**Preparation of target cella**

The positive tumor cell lines (both expressing HLA-A2 and antigen NY-ESO-1) used in the experiment were: A375 and NCI-H1299, and the negative tumor cell lines (expressing only one of HLA-A2 and NY-ESO-1) is: NCI-H1650. The adjusted target cell density was 2 * $10^5$ cells / ml, and 2 * $10^4$ cells / 100 μl was added in each well.

**Preparation of effector cells**

The effector cells were prepared as described in Example 2. The effector cells in this experiment were: Vδ2γδT cells (control) without being transduced with lentivirus (control), Vδ2γδT cells transduced with GFP (control) and Vδ2γδT cells transduced with wild-type (KD = 32μM) 1G4TCR, as well as Vδ2γδT cells transduced with 1G4TCRs of different affinities. The KD values of the high-affinity 1G4 TCR were 4 μM, 1.07 μM, 450 nM, 84 nM, and 5 nM, respectively, and all of the above cells were obtained by expanding a polyclonal cell population obtained by BD Aria flow sorting. The ratio of effector cells to target cells (E: T ratio) was: 10: 1, 5: 1, 1: 1 and 1: 5, respectively.

**Experiment groups were set as follows:**

The experimental group, in which Vδ2γδT cells transduced with NY-ESO-1 specific 1G4TCR mutants of different affinities specifically kill tumor cell lines, was set as follows: 100 μl of target cells was firstly added to a round bottom 96-well plate; and effector cells were also added to the round-bottom 96-well plate in a volume of 100 microliters at 4 effector-to-target ratios.

All of control groups were set as follows: spontaneous LDH experimental well of effector cell: 100 μl of effector cell suspension was added; spontaneous LDH experimental group of target cell: 100 μl of target cell suspension was added; maximum LDH release group of target cell: 100 μl of target cell suspension was added; background control group of medium: only 200μl of medium; volume-corrected control group: only 200 μl of medium. All of experimental wells and control wells were prepared in triplicate, and the final volume of all of wells was 200 μl (supplemented with phenol red-free complete medium).

The plate was placed in a 37°C, 5% $CO_2$ incubator and incubated for 24 hours. 10 μl of lysis solution (10X) per 100μl of medium was added to maximum LDH release well of target cell and volume correction well to lyse the cells; and the plate was incubated in 37°C, 5% humidified $CO_2$ incubator for 45 minutes, centrifuged at 250 xg for 4 minutes. 50 μl of supernatant was taken from each well and transferred to a new 96-well flat-bottom (enzyme analysis) plate. 50 μl of a prepared substrate mixture (12 ml of Assay Buffer was added to a bottle of Substrate Mix and dissolve it at room temperature) was added to each well, and the plate was covered with a tin foil or an opaque box to protect the plate from light. The plate was incubated at room temperature for 30 minutes. 50 μl of stop solution was added to each well to quench the reaction. Absorbance at 490nm was measured within one hour after adding the stop solution, the data were exported and saved in EXCEL format for analyzing results. The counting formula is as follows:

$$\% \text{ Cytotoxicity} = [100 * (\text{experiment - spontaneous effector cell - spontaneous target cell})] / (\text{maximum target cell - spontaneous target cell}).$$

Note: for the experimental well, different effect target ratios (mean) - medium background (mean); for the target cell spontaneous well, target cell spontaneous well (mean) - medium background (mean); for effector cell spontaneous well, spontaneous effector cells of different proportions (mean) - medium background (mean); and for the highest well of target cell, the highgest target cell (mean) - volume correction control (mean). The final drawing software is GraphPad Prism7.

**Results**

The experimental results are shown in Figure 4, in which the X-axis represents different effect-target ratios, and the Y-axis represents the specific killing efficiency on the target cells after the effector cells and the target cells were incubated together. The results showed that γδT cells transduced with αβ1G4TCRs of different high affinities exhibited strong killing effects on positive tumor cell lines, and the killing efficacy was significantly higher than that of γδT cells transduced with wild-type αβTCR. For example, when the effector-target ratio was 10: 1, the killing percentage of yδT cells transduced with high-affinity αβ1G4TCR, the affinity KD of which was 1.07 μM, on the target cells was 70%, which is at least 2 times of that of yδT cells transduced with wild-type αβ1G4TCR. The cell kill percentage is. For another example, when the effector-target ratio was 5: 1, the killing percentage of yδT cells transduced with high-affinity αβ1G4TCR, the affinity KD of which was 84 nM, on the target cells was 50%, while the killing percentage of yδT cells transduced with wild-type αβ1G4TCR on target cells was less than 30%, and the other was less than 10%.

According to a rough estimate, compared with the killing percentage of the wild type αβTCR on target cells, when the KD value of αβTCR affinity was between 4 μM-5 nM, the killing percentage of transduced γδT cells on target cells at different effector-target ratios was increased at least 1 times, preferably, at least a 2-3 times. Generally, when the affinity of αβTCR was 1.07 μM, the killing efficiency of yδT cells was more excellent.

γδT cells transduced with αβ1G4TCRs of different high affinities exhibited basically no killing effects on negative tumor cell lines. γδT cells that have not been transduced with exogenous αβTCR or γδT cells that have only been transfected with GFP exhibited basically no response to the relevant tumor cell lines.

**Example 7. Luciferase assay**

Luciferase reporter gene refers to a reporter system that uses luciferin as a substrate to detect fireflyluciferase activity. Luciferase can catalyze the conversion of luciferin to oxyluciferin through oxidation, and bioluminescence will be emitted during the oxidation of luciferin. The bioluminescence released during the oxidation of luciferin is measured by a fluorescence analyzer. The energy for the redox reaction is necessarily provided by cells, therefore, we incubated effector cells with specific target cells, which will kill the target cells, thereby reducing the energy of the target cells. And the luminescence when catalyzing the fluorescein substrate will be reduced, which can indirectly reflect the killing ability of effector cells on target cells and can be used as a verification to check the function of lactate dehydrogenase LDH. This method is used to reflect the killing effects on target cells when effector cells and target cells are incubated together.

**Main reagents and materials**

The substrate catalyzed by luciferase is D-luciferin (a potassium ion salt, 150 μg / ml, Cayman Chemical, USA); 96-well plate for experiment: purchased from Corning, Catalog number: 3792; tumor cell line: A549 with luciferase was purchased from ATCC. In this experiment, a cell line which expresses NY-ESO-1 antigen and is of HLA-A0201 was constructed.

**Preparation of target cells**

As target cells, A549 expressing Luciferase, NY-ESO-1 and A0201 were used as positive tumor cells, and wild-type A549 (Luciferase) was used as negative control tumor cells and a control group with only corresponding target cells.

**Preparation of effector cells**

The effector cells were prepared as described in Example 2, including Vδ2T cell group without being transduced with lentivirus, Vδ2T cells transduced with GFP, and Vδ2T cell group transduced with wild-type αβ1G4TCR (KD = 32uM) and Vδ2T cell group transduced with high affinity αβ1G4TCR with KD = 1.07uM.

**Methods**

The effector cells and target cells were resuspended in RPMI1640 + 10% FBS + 1% PS complete medium. The effector cells and target cells were innoculated into a 96-well plate at E: T=5: 1 and $2*10^4$ target cells/well; incubated at 37°C, 5% $CO_2$ for 24 hours; 100 ul of Luciferase substrate was added to each well of the experimental group and the control group in darkness, respectively; the target cell viability in the experimental group and the control group was detected by exciting light at 450 nM using a microplate reader; excel data was derived and the killing efficiency of effector cells was calculated according to the formula. The formula is as follows: killing% = (1-signal of experimental group / maximum signal of target cell)%. The final drawing software is GraphPad Prism7.

**Results**

The detection result is shown in Fig. 5, in which the X-axis represents different effector cell groups, and the Y-axis represents the logarithm of the detection values per ml reaction system when the fluorescein substrate for detection is added. The results showed that in the positive tumor cell line A549, the Vδ2T cell group transduced with high affinity αβ1G4TCR exhibited a significantly lower relative luminescence unit as compared with the Vδ2T cell group transduced with wild-type αβ1G4TCR, indicating that Vδ2T cells transduced with high affinity αβTCR have stronger killing effects. For Vδ2T cells that were not transduced or transduced with GFP, their luminescence units did not decrease. In the negative tumor cell lines, there was no significant decrease in the relative luminescence units of each group, indicating that the killing depends on transduced TCR. This experiment also showed that the results of Luciferase detection and LDH detection were consistent.

**Example 8. Dynamic killing test 0f Vδ2T cells transduced with 1G4TCR of different affinities**

Real-time dynamic live cell imaging analyzer - IncuCyte Zoom, a non-invasive method is used to record the real-time growth status of cells. The killing effects of yδT transduced with TCRs of different affinities can be dynamically compared.

Main reagents and materials

96-well plate: purchased from Corning, Catalog number: 3603;
YO-YO-3 staining: NCI-H1299 lung cancer cells, purchased from ATCC is a wild-type expressing antigen NY-ESO-1, but the HLA type thereof is not A0201;
Real-time dynamic live cell imaging analyzer - IncuCyte Zoom: American Essen Company.

Preparation of target cells

NCI-H1299: The wild type purchased from ATCC was modified in the laboratory of the inventors, and the wild type NCI-H1299 cell line was directly transduced with the HLA-A0201 lentivirus to construct the a positive tumor cell line which expresses NY-ESO-1 antigen and is also of HLA-A0201.

Preparation of effector cells

Effector cells was prepaed as described in Example 2, different groups of effector cells were prepared, including Vδ2T cells without being transduced with 1G4TCR and Vδ2T cells transduced with GFP and Vδ2T cells transduced with 1G4TCRs of different affinities of 32 μM (wild type), 4 μM, 1.07 μM, 450 nM, 84 nM and 5 nM.

Methods

The target cells were digested and centrifuged; resuspended in phenol red-free RPMI1640 + 10% FBS complete medium, and counted, 7 * $10^3$ cells / well / 100 ul. The target cells were evenly spread in a 96-well plate; placed in a 37°C, 5% $CO_2$ incubator and incubated overnight; effector cells were prepared at a E: T = 5: 1, and centrifuged; the old culture medium was discarded and replaced with new phenol red-free RPMI1640 + 10% FBS medium (the medium was mixed with YOYO-3 dye of a final concentration of 10000 *). The effector cells were incubated with the target cells in the experimental group at 3.5 * $10^4$ cells / well / 100 ul. 100 ul of complete medium was added to each well in the control group of target cells or effector cells to ensure that the final concentration of YOYO-3 dye in each well is 10000 *; the plate was placed in the Real-time dynamic live cell imaging analyzer - IncuCyte Zoom dedicated to Incucyte detection; real-time observation was started and pictures were taken; and the test results were processed and the data were exported and analyzed by using IncuCyte ZooM 2016A. The final drawing software is GraphPad Prism7.

Results

Detection results are shown in the FIG. 6, in which the X-axis represents the time in which the effector cells were incubated with the target cells, and the Y-axis represents the level of absorption of YO-YO-3 dye by the dead cells, which was used to measure the killing intensity. From the analysis of killing kinetics, compared with Vδ2T cells transduced with wild-type TCR, Vδ2T cells transduced with high-affinity TCR exhibited significantly faster killing effects on positive tumor cell lines, and the killing efficacy was also significantly enhanced.

The sequences involved in the present invention are shown in the following table:

| SEQ ID NO. | Sequence |
|---|---|
| 1 | Atggagaccctgctgggcctgctgatcctgtggctgcagctccagtgggtgtccagcaagcaggaggtgacc cagatccctgccgCcctgagcgtgcccgagggcgagaacctggtgctgaactgcagcttcaccgactccgcc atctacaacctgcagtggttccggcagGaccccggcaagggcctgaccagcctgctgctgattcaatcctcgc agcgggagcagaccagcggacggctgaacgccagcctgGacaagagcagcggccggagcaccctgtaca tcgccgccagccagcccggcgacagcgccacctacctgtgcgctgtgcggcctAcctctggcggcagctac atccccaccttcggcagaggcaccagcctgatcgtgcacccctacatccagaaccccgaccccgccgtGtac cagctgcgggacagcaagagcagcgacaagtctgtgtgcctgttcaccgacttcgacagccagaccaatgtga gccagagcaAggacagcgacgtgtacatcaccgacaagaccgtgctggacatgcggagcatggacttcaag agcaacagcgccgtggcctggagCaacaagagcgacttcgcctgcgccaacgccttcaacaacagcattatc cccgaggacaccttcttccccagccccgagagcagctgCgacgtgaaactggtggagaagagcttcgagac cgacaccaacctgaacttccagaacctgagcgtgatcggcttcagaatcctgctgctgaaggtggccggattca acctgctgatgaccctgcggctgtggagcagc |
| 2 | METLLGLLILWLQLQWVSSKQEVTQIPAALSVPEGENLVLNCSFTDSAI YNLQWFRQDPGKGLTSLLLIQSSQREQTSGRLNASLDKSSGRSTLYIAA SQPGDSATYLCAVRPTSGGSYIPTFGRGTSLIVHPYIQNPDPAVYQLRD SKSSDKSVCLFTDFDSQTNVSQSKDSDVYITDKTVLDMRSMDFKSNSA VAWSNKSDFACANAFNNSIIPEDTFFPSPESSCDVKLVEKSFETDTNLN FQNLSVIGFRILLLKVAGFNLLMTLRLWSS |

(continued)

| SEQ ID NO. | Sequence |
|---|---|
| 3 | Atgagcatcggcctgctgtgctgcgccgccctgagcctgctgtgggcaggacccgtgaacgccggagtgac<br>ccagacccccaagTtccaggtgctgaaaaccggccagagcatgaccctgcagtgcgcccaggacatgaacc<br>acgagtacatgagctggtatcggcaggAccccggcatgggcctgcggctgatccactactctgtgggcgccg<br>ggatcaccgaccagggcgaggtgcccaacggctacaatgtGagccggagcaccaccgaggacttccccct<br>gcggctgctgagcgctgcccccagccagaccagcgtgtacttctgcgccagcagcTatgtgggcaacaccg<br>gcgagctgttcttcggcgagggctccaggctgaccgtgctggaggacctgaagaacgtgttccccccccgaGg<br>tggccgtgttcgagcccagcgaggccgagatcagccacacccagaaggccacactggtgtgtctggccaccg<br>gcttctaccccgAccacgtggagctgtcctggtgggtgaacggcaaggaggtgcacagcggcgtgtctaccg<br>acccccagcccctgaaggagcagccCgccctgaacgacagccggtactgcctgtcctccagactgagagtg<br>agcgccaccttctggcagaacccccggaaccacttccggtgCcaggtgcagttctacggcctgagcgagaac<br>gacgagtggacccaggaccgggccaagcccgtgacccagattgtgagcgccgagGcctggggcagggcc<br>gactgcggcttcaccagcgagagctaccagcagggcgtgctgagcgccaccatcctgtacgagatcctgctg<br>ggcaaggccaccctgtacgccgtgctggtgtctgccctggtgctgatggctatggtgaagcggaaggacagcc<br>ggggctaa |
| 4 | MSIGLLCCAALSLLWAGPVNAGVTQTPKFQVLKTGQSMTLQCAQDM |
| | NHEYMSWYRQDPGMGLRLIHYSVGAGITDQGEVPNGYNVSRSTTEDF<br>PLRLLSAAPSQTSVYFCASSYVGNTGELFFGEGSRLTVLEDLKNVFPPE<br>VAVFEPSEAEISHTQKATLVCLATGFYPDHVELSWWVNGKEVHSGVS<br>TDPQPLKEQPALNDSRYCLSSRLRVSATFWQNPRNHFRCQVQFYGLSE<br>NDEWTQDRAKPVTQIVSAEAWGRADCGFTSESYQQGVLSATILYEILL<br>GKATLYAVLVSALVLMAMVKRKDSRG |
| 5 | Atggagaccctgctgggcctgctgatcctgtggctgcagctccagtgggtgtccagcaagcaggaggtgacc<br>cagatccctgccgcCctgagcgtgcccgagggcgagaacctggtgctgaactgcagcttcaccgactccgcc<br>atctacaacctgcagtggttccggcaggAccccggcaagggcctgaccagcctgctgctgattcaatcctcgc<br>agcgggagcagaccagcggacggctgaacgccagcctggaCaagagcagcggccggagcaccctgtaca<br>tcgccgccagccagcccggcgacagcgccacctacctgtgcgctgtgcggcctaccTctggcggcagctac<br>atccccaccttcggcagaggcaccagcctgatcgtgcacccctacatccagaaccccgaccccgccgtgtac<br>Cagctgcgggacagcaagagcagcgacaagtctgtgtgcctgttcaccgacttcgacagccagaccaatgtg<br>agccagagcaaggaCagcgacgtgtacatcaccgacaagaccgtgctggacatgcggagcatggacttcaa<br>gagcaacagcgccgtggcctggagcaacAagagcgacttcgcctgcgccaacgccttcaacaacagcattat<br>ccccgaggacaccttcttccccagccccgagagcagctgcgaCgtgaaactggtggagaagagcttcgaga<br>ccgacaccaacctgaacttccagaacctgagcgtgatcggcttcagaatcctgctgctgaaggtggccggattc<br>aacctgctgatgaccctgcggctgtggagcagc |
| 6 | METLLGLLILWLQLQWVSSKQEVTQIPAALSVPEGENLVLNCSFTDSAI<br>YNLQWFRQDPGKGLTSLLLIQSSQREQTSGRLNASLDKSSGRSTLYIAA<br>SQPGDSATYLCAVRPTSGGSYIPTFGRGTSLIVHPYIQNPDPAVYQLRD<br>SKSSDKSVCLFTDFDSQTNVSQSKDSDVYITDKTVLDMRSMDFKSNSA<br>VAWSNKSDFACANAFNNSIIPEDTFFPSPESSCDVKLVEKSFETDTNLN<br>FQNLSVIGFRILLLKVAGFNLLMTLRLWSS |

(continued)

| SEQ ID NO. | Sequence |
|---|---|
| 7 | Atgagcatcggcctgctgtgctgcgccgccctgagcctgctgtgggcaggacccgtgaacgccggagtgac<br>ccagaccccccaaGttccaggtgctgaaaaccggccagagcatgaccctgcagtgcgcccaggacatgaacc<br>acgagtacatgagctggtatcggcaGgaccccggcatgggcctgcggctgatccactactctgtgggcgccg<br>ggaccaccgaccagggcgaggtgcccaacggctacAatgtgagccggagcaccaccgaggacttcccccct<br>gcggctgctgagcgctgcccccagccagaccagcgtgtacttctgcgccAgcagctatctgggcgacaccg<br>gcgagctgttcttcggcgagggctccaggctgaccgtgctggaggacctgaagaacgtgttcCcccccgagg<br>tggccgtgttcgagcccagcgaggccgagatcagccacacccagaaggccacactggtgtgtctggccaccg<br>Gcttctaccccgaccacgtggagctgtcctggtgggtgaacggcaaggaggtgcacagcggcgtgtctaccg<br>acccccagcccCtgaaggagcagcccgccctgaacgacagccggtactgcctgtcctccagactgagagtg<br>agcgccaccttctggcagaacccCcggaaccacttccggtgccaggtgcagttctacggcctgagcgagaac<br>gacgagtggacccaggaccgggccaagcccgtgAcccagattgtgagcgccgaggcctggggcagggcc<br>gactgcggcttcaccagcgagagctaccagcagggcgtgctgagcGccaccatcctgtacgagatcctgctg<br>ggcaaggccaccctgtacgccgtgctggtgtctgccctggtgctgatggctatggtgaagcggaaggacagcc<br>ggggctaa |
| 8 | MSIGLLCCAALSLLWAGPVNAGVTQTPKFQVLKTGQSMTLQCAQDM<br>NHEYMSWYRQDPGMGLRLIHYSVGAGTTDQGEVPNGYNVSRSTTED<br>FPLRLLSAAPSQTSVYFCASSYLGDTGELFFGEGSRLTVLEDLKNVFPP<br>EVAVFEPSEAEISHTQKATLVCLATGFYPDHVELSWWVNGKEVHSGV<br>STDPQPLKEQPALNDSRYCLSSRLRVSATFWQNPRNHFRCQVQFYGLS<br>ENDEWTQDRAKPVTQIVSAEAWGRADCGFTSESYQQGVLSATILYEIL<br>LGKATLYAVLVSALVLMAMVKRKDSRG |
| 9 | Atggagaccctgctgggcctgctgatcctgtggctgcagctgcagtgggtgagctccaagcaggaggtgaca<br>cagatccctgcCgccctgagcgtgccagagggagagaacctggtgctgaattgctcttttaccgacagcgcca<br>tctacaacctgcagtggttccgGcaggatccaggcaagggcctgacctctctgctgctgatccagtctagccag<br>cgggagcagacatctggcagactgaatgccAgcctggacaagtcctctggcagatccaccctgtacatcgca<br>gcctcccagccaggcgattctgccacatatctgtgcgccgtgAggccactgtacggaggaagctatatcccta<br>cctttggccgcggcacatccctgatcgtgcacccttacatccagaacccagacCccgccgtgtatcagctgag |
| | ggactccaagagctccgataagagcgtgtgcctgttcaccgactttgattctcagacaaacgtgaGccagtcta<br>aggacagcgacgtgtacatcaccgacaagacagtgctggatatgcgctccatggacttcaagagcaactccgc<br>cgTggcctggtctaataagagcgatttcgcctgcgccaacgcctttaacaattccatcatccctgaggataccttc<br>tttccttctccagagTctagctgtgacgtgaagctggtggagaagtccttcgagaccgatacaaacctgaattttc<br>agaacctgtctgtgatcggcttcaggatcctgctgctgaaggtggccggctttaatctgctgatgaccctgaggct<br>gtggtcctct |
| 10 | METLLGLLILWLQLQWVSSKQEVTQIPAALSVPEGENLVLNCSFTDSAI<br>YNLQWFRQDPGKGLTSLLLIQSSQREQTSGRLNASLDKSSGRSTLYIAA<br>SQPGDSATYLCAVRPLYGGSYIPTFGRGTSLIVHPYIQNPDPAVYQLRD<br>SKSSDKSVCLFTDFDSQTNVSQSKDSDVYITDKTVLDMRSMDFKSNSA<br>VAWSNKSDFACANAFNNSIIPEDTFFPSPESSCDVKLVEKSFETDTNLN<br>FQNLSVIGFRILLLKVAGFNLLMTLRLWSS |

(continued)

| SEQ ID NO. | Sequence |
|---|---|
| 11 | AtgagcatcggactgctgtgctgtgccgccctgtccctgctgtgggcaggacctgtgaacgcaggagtgacccagacaccaaAgtttcaggtgctgaagaccggccagtccatgacactgcagtgcgcccaggacatgaatcacgagtacatgtcttggtataggCaggatccaggaatgggactgaggctgatccactacagcgtgggagcaggaatcacagaccagggagaggtgcctaacggCtataacgtgagccggtctaccacagaggattttccactgagactgctgagcgccgcaccatcccagaccagcgtgtacttctgCgccagctcctatgtgggcaacaccggcgagctgttctttggagagggatcccggctgacagtgctggaggacctgaagaacGtgttcccccctgaggtggccgtgtttgagccaagcgaggccgagatctcccacacccagaaggccaccctggtgtgcctggcCacaggcttctaccccgatcacgtggagctgagctggtgggtgaacggcaaggaggtgcacagcggcgtgtccaccgacccaCagcccctgaaggagcagcctgccctgaatgattctagatactgcctgtctagccggctgagagtgagcgccaccttttggcagAacccacggaatcacttcagatgtcaggtgcagttttatggcctgagcgagaacgatgagtggacccaggacagggcaaagccAgtgacacagatcgtgtccgccgaggcatggggaagagcagactgtggcttcaccagcgagtcctatcagcagggcgtgctgtCcgccaccatcctgtacgagatcctgctgggcaaggccacactgtatgccgtgctggtgtctgccctggtgctgatggccatggtgaagaggaaggatagccgcggctaa |
| 12 | MSIGLLCCAALSLLWAGPVNAGVTQTPKFQVLKTGQSMTLQCAQDMNHEYMSWYRQDPGMGLRLIHYSVGAGITDQGEVPNGYNVSRSTTEDFPLRLLSAAPSQTSVYFCASSYVGNTGELFFGEGSRLTVLEDLKNVFPPEVAVFEPSEAEISHTQKATLVCLATGFYPDHVELSWWVNGKEVHSGVSTDPQPLKEQPALNDSRYCLSSRLRVSATFWQNPRNHFRCQVQFYGLSENDEWTQDRAKPVTQIVSAEAWGRADCGFTSESYQQGVLSATILYEILLGKATLYAVLVSALVLMAMVKRKDSRG |
| 13 | AtggagaccctgctgggcctgctgatcctgtggctgcagctccagtgggtgtccagcaagcaggaggtgacccagatccctgccGccctgagcgtgcccgagggcgagaacctggtgctgaactgcagcttcaccgactccgccatctacaacctgcagtggttccggCaggaccccggcaagggcctgaccagcctgctgctgattcaatcctcgcagcgggagcagaccagcggacggctgaacgccaGcctggacaagagcagcggccggagcaccctgtacatcgccgccagccagcccggcgacagcgccacctacctgtgcgctgtGcggcctctgacaggcggcagctacatccccaccttcggcagaggcaccagcctgatcgtgcacccctacatccagaaccccgAccccgccgtgtaccagctgcgggacagcaagagcagcgacaagtctgtgtgcctgttcaccgacttcgacagccagaccaatgTgagccagagcaaggacagcgacgtgtacatcaccgacaagaccgtgctggacatgcggagcatggacttcaagagcaacagcGccgtggcctggagcaacaagagcgacttcgcctgcgccaacgccttcaacaacagcattatccccgaggacaccttcttccccaGccccgagagcagctgcgacgtgaaactggtggagaagagcttcgagaccgacaccaacctgaacttccagaacctgagcgtgatcggcttcagaatcctgctgctgaaggtggccggattcaacctgctgatgaccctgcggctgtggagcagc |
| 14 | METLLGLLILWLQLQWVSSKQEVTQIPAALSVPEGENLVLNCSFTDSAIYNLQWFRQDPGKGLTSLLLIQSSQREQTSGRLNASLDKSSGRSTLYIAASQPGDSATYLCAVRPLTGGSYIPTFGRGTSLIVHPYIQNPDPAVYQLRDSKSSDKSVCLFTDFDSQTNVSQSKDSDVYITDKTVLDMRSMDFKSNSAVAWSNKSDFACANAFNNSIIPEDTFFPSPESSCDVKLVEKSFETDTNLNFQNLSVIGFRILLLKVAGFNLLMTLRLWSS |
| 15 | Atgagcatcggcctgctgtgctgcgccgccctgagcctgctgtgggcaggacccgtgaacgccggagtgac |

(continued)

| SEQ ID NO. | Sequence |
|---|---|
| | ccagaccccca Agttccaggtgctgaaaaccggccagagcatgaccctgcagtgcgcccaggacatgaacc acgagtacatgagctggtatcggCaggaccccggcatgggcctgcggctgatccactactctgtgggcgccg ggaccaccgaccagggcgaggtgcccaacggctAcaatgtgagccggagcaccaccgaggacttccccct gcggctgctgagcgctgcccccagccagaccagcgtgtacttctgcgCcagcagcaatgtgggcaacaccg gcgagctgttcttcggcgagggctccaggctgaccgtgctggaggacctgaagaacgtgtTcccccccgagg tggccgtgttcgagcccagcgaggccgagatcagccacacccagaaggccacactggtgtgtctggccacc Ggcttctaccccgaccacgtggagctgtcctggtgggtgaacggcaaggaggtgcacagcggcgtgtctacc gacccccagcccCtgaaggagcagcccgccctgaacgacagccggtactgcctgtcctccagactgagagt gagcgccaccttctggcagaaccccCggaaccacttccggtgccaggtgcagttctacggcctgagcgagaa cgacgagtggacccaggaccgggccaagcccgtgacCcagattgtgagcgccgaggcctggggcagggc cgactgcggcttcaccagcgagagctaccagcagggcgtgctgagcgccAccatcctgtacgagatcctgct gggcaaggccaccctgtacgccgtgctggtgtctgccctggtgctgatggctatggtgaagcggaaggacag ccggggctaa |
| 16 | MSIGLLCCAALSLLWAGPVNAGVTQTPKFQVLKTGQSMTLQCAQDM NHEYMSWYRQDPGMGLRLIHYSVGAGTTDQGEVPNGYNVSRSTTED FPLRLLSAAPSQTSVYFCASSNVGNTGELFFGEGSRLTVLEDLKNVFPP EVAVFEPSEAEISHTQKATLVCLATGFYPDHVELSWWVNGKEVHSGV STDPQPLKEQPALNDSRYCLSSRLRVSATFWQNPRNHFRCQVQFYGLS ENDEWTQDRAKPVTQIVSAEAWGRADCGFTSESYQQGVLSATILYEIL LGKATLYAVLVSALVLMAMVKRKDSRG |
| 17 | Atggagaccctgctgggcctgctgatcctgtggctgcagctccagtgggtgtccagcaagcaggaggtgacc cagatccctgCcgccctgagcgtgcccgagggcgagaacctggtgctgaactgcagcttcaccgactccgcc atctacaacctgcagtggttCcggcaggaccccggcaagggcctgaccagcctgctgctgattcaatcctcgc agcgggagcagaccagcggacggctgAacgccagcctggacaagagcagcggccggagcaccctgtac atcgccgccagccagcccggcgacagcgccacctaccTgtgcgctgtgcggcctatgattggcggcaccta catccccaccttcggcagaggcaccagcctgatcgtgcacccctacatccAgaaccccgaccccgccgtgta ccagctgcgggacagcaagagcagcgacaagtctgtgtgcctgttcaccgacttcgacagCcagaccaatgt gagccagagcaaggacagcgacgtgtacatcaccgacaagaccgtgctggacatgcggagcatggacttcA agagcaacagcgccgtggcctggagcaacaagagcgacttcgcctgcgccaacgccttcaacaacagcatta tccccgaggAcaccttcttccccagccccgagagcagctgcgacgtgaaactggtggagaagagcttcgaga ccgacaccaacctgaacttcCagaacctgagcgtgatcggcttcagaatcctgctgctgaaggtggccggatt caacctgctgatgaccctgcggctgtggagcagc |
| 18 | METLLGLLILWLQLQWVSSKQEVTQIPAALSVPEGENLVLNCSFTDSAI YNLQWFRQDPGKGLTSLLLIQSSQREQTSGRLNASLDKSSGRSTLYIAA SQPGDSATYLCAVRPMIGGTYIPTFGRGTSLIVHPYIQNPDPAVYQLRD SKSSDKSVCLFTDFDSQTNVSQSKDSDVYITDKTVLDMRSMDFKSNSA VAWSNKSDFACANAFNNSIIPEDTFFPSPESSCDVKLVEKSFETDTNLN FQNLSVIGFRILLLKVAGFNLLMTLRLWSS |

(continued)

| SEQ ID NO. | Sequence |
|---|---|
| 19 | Atgagcatcggcctgctgtgctgcgccgccctgagcctgctgtgggcaggacccgtgaacgccggagtgac ccagacccccAagttccaggtgctgaaaaccggccagagcatgaccctgcagtgcgcccaggacatgaacc acgagtacatgagctggtatcGgcaggaccccggcatgggcctgcggctgatccactactctgtgggcgccc agaccaccgaccagggcgaggtgcccaacGgctacaatgtgagccggagcaccatcgaggacttccccctg cggctgctgagcgctgcccccagccagaccagcgtgtactTctgcgccagcagctatctgggcaacaccgg cgagctgttcttcggcgagggctccaggctgaccgtgctggaggacctgaaGaacgtgttccccccgaggt ggccgtgttcgagcccagcgaggccgagatcagccacacccagaaggccacactggtgtgTctggccacc ggcttctaccccgaccacgtggagctgtcctggtgggtgaacggcaaggaggtgcacagcggcgtgtctacc Gacccccagcccctgaaggagcagcccgccctgaacgacagccggtactgcctgtcctccagactgagagt gagcgccaccTtctggcagaacccccggaaccacttccggtgccaggtgcagttctacggcctgagcgaga acgacgagtggacccaggaccGggccaagcccgtgacccagattgtgagcgccgaggcctggggcaggg ccgactgcggcttcaccagcgagagctaccagCagggcgtgctgagcgccaccatcctgtacgagatcctgc tgggcaaggccaccctgtacgccgtgctggtgtctgccctggtgctgatggctatggtgaagcggaaggacag |
| | ccggggctaa |
| 20 | MSIGLLCCAALSLLWAGPVNAGVTQTPKFQVLKTGQSMTLQCAQDM NHEYMSWYRQDPGMGLRLIHYSVGAQTTDQGEVPNGYNVSRSTIEDF PLRLLSAAPSQTSVYFCASSYLGNTGELFFGEGSRLTVLEDLKNVFPPE VAVFEPSEAEISHTQKATLVCLATGFYPDHVELSWWVNGKEVHSGVS TDPQPLKEQPALNDSRYCLSSRLRVSATFWQNPRNHFRCQVQFYGLSE NDEWTQDRAKPVTQIVSAEAWGRADCGFTSESYQQGVLSATILYEILL GKATLYAVLVSALVLMAMVKRKDSRG |
| 21 | atggagaccctgctgggcctgctgatcctgtggctgcagctccagtgggtgtccagcaagcaggaggtgaccc agatccctgccgccctgagcgtgcccgagggcgagaacctggtgctgaactgcagcttcaccgactccgccat ctacaacctgcagtggttccggcaggaccccggcaagggcctgaccagcctgctgctgattcaatcctcgcag cgggagcagaccagcggacggctgaacgccagcctggacaagagcagcggccggagcaccctgtacatc gccgccagccagcccggcgacagcgccacctacctgtgcgctgtgcggcctctgctggacggcacctacatc cccaccttcggcagaggcaccagcctgatcgtgcacccctacatccagaaccccgaccccgccgtgtaccag ctgcgggacagcaagagcagcgacaagtctgtgtgcctgttcaccgacttcgacagccagaccaatgtgagcc agagcaaggacagcgacgtgtacatcaccgacaagaccgtgctggacatgcggagcatggacttcaagagc aacagcgccgtggcctggagcaacaagagcgacttcgcctgcgccaacgccttcaacaacagcattatcccc gaggacaccttcttccccagccccgagagcagctgcgacgtgaaactggtggagaagagcttcgagaccgac accaacctgaacttccagaacctgagcgtgatcggcttcagaatcctgctgctgaaggtggccggattcaacct gctgatgaccctgcggctgtggagcagc |
| 22 | METLLGLLILWLQLQWVSSKQEVTQIPAALSVPEGENLVLNCSFTDSAI YNLQWFRQDPGKGLTSLLLIQSSQREQTSGRLNASLDKSSGRSTLYIAA SQPGDSATYLCAVRPLLDGTYIPTFGRGTSLIVHPYIQNPDPAVYQLRD SKSSDKSVCLFTDFDSQTNVSQSKDSDVYITDKTVLDMRSMDFKSNSA VAWSNKSDFACANAFNNSIIPEDTFFPSPESSCDVKLVEKSFETDTNLN FQNLSVIGFRILLLKVAGFNLLMTLRLWSS |

(continued)

| SEQ ID NO. | Sequence |
|---|---|
| 23 | atgagcatcggcctgctgtgctgcgccgccctgagcctgctgtgggcaggacccgtgaacgccggagtgacc<br>cagacccccaagttccaggtgctgaaaaccggccagagcatgaccctgcagtgcgcccaggacatgaacca<br>cgagtacatgagctggtatcggcaggaccccggcatgggcctgcggctgatccactactctgtgggcgccgg<br>gaccaccgaccgcggcgaggtgcccaacggctacaatgtgagccggagcaccatcgaggacttccccctgc<br>ggctgctgagcgctgcccccagccagaccagcgtgtacttctgcgccagcagctatgtgggcgacaccggcg<br>agctgttcttcggcgagggctccaggctgaccgtgctggaggacctgaagaacgtgttcccccccgaggtggc<br>cgtgttcgagcccagcgaggccgagatcagccacacccagaaggccacactggtgtgtctggccaccggctt<br>ctaccccgaccacgtggagctgtcctggtgggtgaacggcaaggaggtgcacagcggcgtgtctaccgaccc<br>ccagcccctgaaggagcagcccgccctgaacgacagccggtactgcctgtcctccagactgagagtgagcg<br>ccaccttctggcagaacccccggaaccacttccggtgccaggtgcagttctacggcctgagcgagaacgacg<br>agtggacccaggaccgggccaagcccgtgacccagattgtgagcgccgaggcctggggcagggccgactg<br>cggcttcaccagcgagagctaccagcagggcgtgctgagcgccaccatcctgtacgagatcctgctgggcaa<br>ggccaccctgtacgccgtgctggtgtctgccctggtgctgatggctatggtgaagcggaaggacagccgggg<br>ctaa |
| 24 | MSIGLLCCAALSLLWAGPVNAGVTQTPKFQVLKTGQSMTLQCAQDM<br>NHEYMSWYRQDPGMGLRLIHYSVGAGTTDRGEVPNGYNVSRSTIEDF<br>PLRLLSAAPSQTSVYFCASSYVGDTGELFFGEGSRLTVLEDLKNVFPPE<br>VAVFEPSEAEISHTQKATLVCLATGFYPDHVELSWWVNGKEVHSGVS<br>TDPQPLKEQPALNDSRYCLSSRLRVSATFWQNPRNHFRCQVQFYGLSE<br>NDEWTQDRAKPVTQIVSAEAWGRADCGFTSESYQQGVLSATILYEILL<br>GKATLYAVLVSALVLMAMVKRKDSRG |

All documents mentioned in the present invention are cited as references in this application, as if each document is individually cited as a reference. In addition, it should be understood that after reading the above teachings of the present invention, a skilled person in the art can make various changes or modifications to the present invention, and these equivalent forms also fall within the scope defined by the appended claims of the present application.

## Claims

1. A genetically modified γδT cell, into which a gene of high-affinity αβTCR has been transduced, and the affinity of the high-affinity αβTCR to its specific pMHC is at least two times that of the corresponding wild-type αβTCR.

2. The γδT cell of claim 1, wherein the equilibrium dissociation constant value KD of the affinity of the high-affinity αβ TCR to its specific pMHC is ≤ 10 μM.

3. The γδT cell of claim 1, wherein the equilibrium dissociation constant value KD of the affinity of the high-affinity αβTCR to its specific pMHC is ≤ 4 μM; for example, 5 nM ≤ KD ≤ 4000 nM, 50 nM ≤ KD ≤ 4000 nM, 100 nM ≤ KD ≤ 4000 nM, 400 nM ≤ KD ≤ 4000 nM, 450 nM ≤ KD ≤ 4000 nM, 400 nM ≤ KD ≤ 3000 nM, 500 nM ≤ KD ≤ 3000 nM, 1000 nM ≤ KD ≤ 3000 nM or 1500 nM ≤ KD ≤ 3000 nM.

4. The γδT cell of claim 1, wherein the equilibrium dissociation constant value KD of the affinity of the high affinity αβ TCR to its specific pMHC is: 1 nM ≤ KD ≤ 2000 nM; for example, 100 nM ≤ KD ≤ 2000 nM, 400 nM ≤ KD ≤ 2000 nM, 450 nM ≤ KD ≤ 2000 nM, 500 nM ≤ KD ≤ 2000 nM, 1000 nM ≤ KD ≤ 2000 nM or 1500 nM ≤ KD ≤ 2000 nM.

5. The γδT cell of any one of claims 1-4, wherein the γδT cells are used to prepare medicaments for treating a tumor or an infectious disease.

6. The γδT cell of claim 5, wherein the infection is a viral infection or a bacterial infection.

7. Use of the γδT cell of any one of claims 1-6 for preparing a medicament for treating a tumor or infectious disease.

8. A pharmaceutical composition, wherein the pharmaceutical composition comprises a pharmaceutically acceptable carrier and the yδ T cell of any one of claims 1-6.

9. A method for treating a disease, comprising administering to a subject in need thereof an appropriate amount of the yδ T cell of any one of claims 1-6 or the pharmaceutical composition of claim 8.

10. A method for preparing the γδT cell of any one of claims 1-6, the method comprising the following steps:

(i) transducing a gene of high-affinity αβTCR into a γδT cell;
(ii) culturing the γδT cell described in (i).

Nucleic acid sequence SEQ ID NO:1

atggagaccctgctgggcctgctgatcctgtggctgcagctccagtgggtgtccagcaagcaggaggtgacccagatccctgccgccctgagcgtgcccgagggcgagaacct
ggtgctgaactgcagcttcaccgactccgccatctacaacctgcagtggttccggcaggaccccggcaagggcctgaccagcctgctgctgattcaatcctcgcagcgggagca
gaccagcggacggctgaacgccagcctggacaagagcagcggccggagcaccctgtacatcgccgccagccagcccggcgacagcgccacctacctgtgcgctgtgcggc
ctacctctggcggcagctacatccccaccttcggcagaggcaccagcctgatcgtgcacccctacatccagaaccccgaccccgccgtgtaccagctgcgggacagcaagagc
agcgacaagtctgtgtgcctgttcaccgacttcgacagccagaccaatgtgagccagagcaaggacagcgacgtgtacatcaccgacaagaccgtgctggacatgcggagcat
ggacttcaagagcaacagcgccgtggcctggagcaacaagagcgacttcgcctgcgccaacgccttcaacaacagcattatccccgaggacaccttcttccccagccccgaga
gcagctgcgacgtgaaactggtggagaagagcttcgagaccgacaccaacctgaacttccagaacctgagcgtgatcggcttcagaatcctgctgctgaaggtggccggattca
acctgctgatgaccctgcggctgtggagcagc

Fig. 1a

amino acid sequence SEQ ID NO:2

METLLGLLILWLQLQWVSSKQEVTQIPAALSVPEGENLVLNCSFTDSAIYNLQWFRQDPGKGLTSLLLIQSSQREQTS
GRLNASLDKSSGRSTLYIAASQPGDSATYLCAVRPTSGGSYIPTFGRGTSLIVHPYIQNPDPAVYQLRDSKSSDKSVC
LFTDFDSQTNVSQSKDSDVYITDKTVLDMRSMDFKSNSAVAWSNKSDFACANAFNNSIIPEDTFFPSPESSCDVKLV
EKSFETDTNLNFQNLSVIGFRILLLKVAGFNLLMTLRLWSS

Fig. 1b

Nucleic acid sequence SEQ ID NO:3

atgagcatcggcctgctgtgctgcgccgccctgagcctgctgtgggcaggacccgtgaacgccggagtgacccagacccccaagttccaggtgctgaaaaccggccagagca
tgaccctgcagtgcgcccaggacatgaaccacgagtacatgagctggtatcggcaggaccccggcatgggcctgcggctgatccactactctgtgggcgccgggatcaccgac
cagggcgaggtgcccaacggctacaatgtgagccggagcaccaccgaggacttcccctgcggctgctgagcgctgcccccagccagaccagcgtgtacttctgcgccagca
gctatgtgggcaacaccggcgagctgttcttcggcgagggctccaggctgaccgtgctggaggacctgaagaacgtgttcccccccgaggtggccgtgttcgagcccagcgag
gccgagatcagccacacccagaaggccacactggtgtgtctggccaccggcttctaccccgaccacgtggagctgtcctggtgggtgaacggcaaggaggtgcacagcggcg
tgtctaccgacccccagcccctgaaggagcagcccgccctgaacgacagccggtactgcctgtcctccagactgagagtgagcgccaccttctggcagaacccccggaaccac
ttccggtgccaggtgcagttctacggcctgagcgagaacgacgagtggacccaggaccgggccaagcccgtgacccagattgtgagcgccgaggcctggggcagggccgac
tgcggcttcaccagcgagagctaccagcagggcgtgctgagcgccaccatcctgtacgagatcctgctgggcaaggccaccctgtacgccgtgctggtgtctgccctggtgctg
atggctatggtgaagcggaaggacagccggggctaa

Fig. 1c

amino acid sequence SEQ ID NO:4

MSIGLLCCAALSLLWAGPVNAGVTQTPKFQVLKTGQSMTLQCAQDMNHEYMSWYRQDPGMGLRLIHYSVGAGIT
DQGEVPNGYNVSRSTTEDFPLRLLSAAPSQTSVYFCASSYVGNTGELFFGEGSRLTVLEDLKNVFPPEVAVFEPSEAE
ISHTQKATLVCLATGFYPDHVELSWWVNGKEVHSGVSTDPQPLKEQPALNDSRYCLSSRLRVSATFWQNPRNHFRC
QVQFYGLSENDEWTQDRAKPVTQIVSAEAWGRADCGFTSESYQQGVLSATILYEILLGKATLYAVLVSALVLMAM
VKRKDSRG.

Fig. 1d

Nucleic acid sequence SEQ ID NO:5

atggagaccctgctgggcctgctgatcctgtggctgcagctccagtgggtgtccagcaagcaggaggtgacccagatccctgccgccctgagcgtgcccgagggcgagaacct
ggtgctgaactgcagcttcaccgactccgccatctacaacctgcagtggttccggcaggaccccggcaagggcctgaccagcctgctgctgattcaatcctcgcagcgggagca
gaccagcggacggctgaacgccagcctggacaagagcagcggccggagcaccctgtacatcgccgccagccagcccggcgacagcgccacctacctgtgcgctgtgcggc
ctacctctggcggcagctacatccccaccttcggcagaggcaccagcctgatcgtgcacccctacatccagaaccccgaccccgccgtgtaccagctgcgggacagcaagagc
agcgacaagtctgtgtgcctgttcaccgacttcgacagccagaccaatgtgagccagagcaaggacagcgacgtgtacatcaccgacaagaccgtgctggacatgcggagcat
ggacttcaagagcaacagcgccgtggcctggagcaacaagagcgacttcgcctgcgccaacgccttcaacaacagcattatccccgaggacaccttcttccccagccccgaga
gcagctgcgacgtgaaactggtggagaagagcttcgagaccgacaccaacctgaacttccagaacctgagcgtgatcggcttcagaatcctgctgctgaaggtggccggattca
acctgctgatgaccctgcggctgtggagcagc

Fig. 1e

amino acid sequence SEQ ID NO:6

METLLGLLILWLQLQWVSSKQEVTQIPAALSVPEGENLVLNCSFTDSAIYNLQWFRQDPGKGLTSLLLIQSSQREQTS
GRLNASLDKSSGRSTLYIAASQPGDSATYLCAVRPTSGGSYIPTFGRGTSLIVHPYIQNPDPAVYQLRDSKSSDKSVC
LFTDFDSQTNVSQSKDSDVYITDKTVLDMRSMDFKSNSAVAWSNKSDFACANAFNNSIIPEDTFFPSPESSCDVKLV
EKSFETDTNLNFQNLSVIGFRILLLKVAGFNLLMTLRLWSS

Fig. 1f

Nucleic acid sequence SEQ ID NO:7

atgagcatcggcctgctgtgctgcgccgccctgagcctgctgtgggcaggacccgtgaacgccggagtgacccagacccccaagttccaggtgctgaaaaccggccagagca
tgaccctgcagtgcgcccaggacatgaaccacgagtacatgagctggtatcggcaggaccccggcatgggcctgcggctgatccactactctgtgggcgccgggaccaccga
ccagggcgaggtgcccaacggctacaatgtgagccggagcaccaccgaggacttcccctgcggctgctgagcgctgcccccagccagaccagcgtgtacttctgcgccagc
agctatctgggcgacaccggcgagctgttcttcggcgagggctccaggctgaccgtgctggaggacctgaagaacgtgttcccccccgaggtggccgtgttcgagcccagcga
ggccgagatcagccacacccagaaggccacactggtgtgtctggccaccggcttctaccccgaccacgtggagctgtcctggtgggtgaacggcaaggaggtgcacagcggc
gtgtctaccgacccccagcccctgaaggagcagcccgccctgaacgacagccggtactgcctgtcctccagactgagagtgagcgccaccttctggcagaacccccggaacc
acttccggtgccaggtgcagttctacggcctgagcgagaacgacgagtggacccaggaccgggccaagcccgtgacccagattgtgagcgccgaggcctggggcagggccg
actgcggcttcaccagcgagagctaccagcagggcgtgctgagcgccaccatcctgtacgagatcctgctgggcaaggccaccctgtacgccgtgctggtgtctgccctggtgc
tgatggctatggtgaagcggaaggacagccggggctaa

Fig. 1g

amino acid sequence SEQ ID NO:8
MSIGLLCCAALSLLWAGPVNAGVTQTPKFQVLKTGQSMTLQCAQDMNHEYMSWYRQDPGMGLRLIHYSVGAGTT
DQGEVPNGYNVSRSTTEDFPLRLLSAAPSQTSVYFCASSYLGDTGELFFGEGSRLTVLEDLKNVFPPEVAVFEPSEAE
ISHTQKATLVCLATGFYPDHVELSWWVNGKEVHSGVSTDPQPLKEQPALNDSRYCLSSRLRVSATFWQNPRNHFRC
QVQFYGLSENDEWTQDRAKPVTQIVSAEAWGRADCGFTSESYQQGVLSATILYEILLGKATLYAVLVSALVLMAM
VKRKDSRG.

Fig. 1h

Nucleic acid sequence SEQ ID NO:9
atggagaccctgctgggcctgctgatcctgtggctgcagctgcagtgggtgagctccaagcaggaggtgacacagatccctgccgccctgagcgtgccagagggagagaacct
ggtgctgaattgctcttttaccgacagcgccatctacaacctgcagtggttccggcaggatccaggcaagggcctgacctctctgctgctgatccagtctagccagcgggagcag
acatctggcagactgaatgccagcctggacaagtcctctggcagatccaccctgtacatcgcagcctcccagccaggcgattctgccacatatctgtgcgccgtgaggccactgt
acggaggaagctatatccctacctttggccgcggcacatccctgatcgtgcacccttacatccagaacccagaccccgccgtgtatcagctgagggactccaagagctccgataa
gagcgtgtgcctgttcaccgactttgattctcagacaaacgtgagccagtctaaggacagcgacgtgtacatcaccgacaagacagtgctggatatgcgctccatggacttcaaga
gcaactccgccgtggcctggtctaataagagcgatttcgcctgcgccaacgcctttaacaattccatcatccctgaggataccttctttccttctccagagtctagctgtgacgtgaag
ctggtggagaagtccttcgagaccgatacaaacctgaattttcagaacctgtctgtgatcggcttcaggatcctgctgctgaaggtggccggctttaatctgctgatgaccctgaggc
tgtggtcctct

Fig. 1i

amino acid sequence SEQ ID NO:10
METLLGLLILWLQLQWVSSKQEVTQIPAALSVPEGENLVLNCSFTDSAIYNLQWFRQDPGKGLTSLLLIQSSQREQTS
GRLNASLDKSSGRSTLYIAASQPGDSATYLCAVRPLYGGSYIPTFGRGTSLIVHPYIQNPDPAVYQLRDSKSSDKSVC
LFTDFDSQTNVSQSKDSDVYITDKTVLDMRSMDFKSNSAVAWSNKSDFACANAFNNSIIPEDTFFPSPESSCDVKLV
EKSFETDTNLNFQNLSVIGFRILLLKVAGFNLLMTLRLWSS

Fig. 1j

Nucleic acid sequence SEQ ID NO:11
atgagcatcggactgctgtgctgtgccgccctgtccctgctgtgggcaggacctgtgaacgcaggagtgacccagacaccaaagtttcaggtgctgaagaccggccagtccatg
acactgcagtgcgcccaggacatgaatcacgagtacatgtcttggtataggcaggatccaggaatgggactgaggctgatccactacagcgtgggagcaggaatcacagacca
gggagaggtgcctaacggctataacgtgagccggtctaccacagaggattttccactgagactgctgagcgccgcaccatcccagaccagcgtgtacttctgcgccagctcctat
gtgggcaacaccggcgagctgttctttggagagggatcccggctgacagtgctggaggacctgaagaacgtgttccccccctgaggtggccgtgtttgagccaagcgaggccga
gatctcccacacccagaaggccaccctggtgtgcctggccacaggcttctaccccgatcacgtggagctgagctggtgggtgaacggcaaggaggtgcacagcggcgtgtcca
ccgacccacagcccctgaaggagcagcctgccctgaatgattctagatactgcctgtctagccggctgagagtgagcgccaccttttggcagaacccacggaatcacttcagatg
tcaggtgcagttttatggcctgagcgagaacgatgagtggacccaggacagggcaaagccagtgacacagatcgtgtccgccgaggcatggggaagagcagactgtggcttca
ccagcgagtcctatcagcagggcgtgctgtccgccaccatcctgtacgagatcctgctgggcaaggccacactgtatgccgtgctggtgtctgccctggtgctgatggccatggt
gaagaggaaggatagccgcggctaa

Fig. 1k

amino acid sequence SEQ ID NO:12
MSIGLLCCAALSLLWAGPVNAGVTQTPKFQVLKTGQSMTLQCAQDMNHEYMSWYRQDPGMGLRLIHYSVGAGIT
DQGEVPNGYNVSRSTTEDFPLRLLSAAPSQTSVYFCASSYVGNTGELFFGEGSRLTVLEDLKNVFPPEVAVFEPSEAE
ISHTQKATLVCLATGFYPDHVELSWWVNGKEVHSGVSTDPQPLKEQPALNDSRYCLSSRLRVSATFWQNPRNHFRC
QVQFYGLSENDEWTQDRAKPVTQIVSAEAWGRADCGFTSESYQQGVLSATILYEILLGKATLYAVLVSALVLMAM
VKRKDSRG.

Fig. 1l

Nucleic acid sequence SEQ ID NO:13
atggagaccctgctgggcctgctgatcctgtggctgcagctccagtgggtgtccagcaagcaggaggtgacccagatccctgccgccctgagcgtgcccgagggcgagaacct
ggtgctgaactgcagcttcaccgactccgccatctacaacctgcagtggttccggcaggaccccggcaagggcctgaccagcctgctgctgattcaatcctcgcagcgggagca
gaccagcggacggctgaacgccagcctggacaagagcagcggccggagcaccctgtacatcgccgccagccagcccggcgacagcgccacctacctgtgcgctgtgcggc
ctctgacaggcggcagctacatccccaccttcggcagaggcaccagcctgatcgtgcacccctacatccagaaccccgaccccgccgtgtaccagctgcgggacagcaagag
cagcgacaagtctgtgtgcctgttcaccgacttcgacagccagaccaatgtgagccagagcaaggacagcgacgtgtacatcaccgacaagaccgtgctggacatgcggagca
tggacttcaagagcaacagcgccgtggcctggagcaacaagagcgacttcgcctgcgccaacgccttcaacaacagcattatccccgaggacaccttcttccccagccccgag
agcagctgcgacgtgaaactggtggagaagagcttcgagaccgacaccaacctgaacttccagaacctgagcgtgatcggcttcagaatcctgctgctgaaggtggccggattc
aacctgctgatgaccctgcggctgtggagcagc

Fig. 1m

amino acid sequence SEQ ID NO:14
METLLGLLILWLQLQWVSSKQEVTQIPAALSVPEGENLVLNCSFTDSAIYNLQWFRQDPGKGLTSLLLIQSSQREQTS
GRLNASLDKSSGRSTLYIAASQPGDSATYLCAVRPLTGGSYIPTFGRGTSLIVHPYIQNPDPAVYQLRDSKSSDKSVC
LFTDFDSQTNVSQSKDSDVYITDKTVLDMRSMDFKSNSAVAWSNKSDFACANAFNNSIIPEDTFFPSPESSCDVKLV
EKSFETDTNLNFQNLSVIGFRILLLKVAGFNLLMTLRLWSS

Fig. 1n

Nucleic acid sequence SEQ ID NO:15
atgagcatcggcctgctgtgctgcgccgccctgagcctgctgtgggcaggacccgtgaacgccggagtgacccagacccccaagttccaggtgctgaaaaccggccagagca
tgaccctgcagtgcgcccaggacatgaaccacgagtacatgagctggtatcggcaggaccccggcatgggcctgcggctgatccactactctgtgggcgccgggaccaccga
ccagggcgaggtgcccaacggctacaatgtgagccggagcaccaccgaggacttccccctgcggctgctgagcgctgcccccagccagaccagcgtgtacttctgcgccagc
agcaatgtgggcaacaccggcgagctgttcttcggcgagggctccaggctgaccgtgctggaggacctgaagaacgtgttccccccgaggtggccgtgttcgagcccagcga
ggccgagatcagccacacccagaaggccacactggtgtgtctggccaccggcttctaccccgaccacgtggagctgtcctggtgggtgaacggcaaggaggtgcacagcggc
gtgtctaccgacccccagcccctgaaggagcagcccgccctgaacgacagccggtactgcctgtcctccagactgagagtgagcgccaccttctggcagaacccccggaacc
acttccggtgccaggtgcagttctacggcctgagcgagaacgacgagtggacccaggaccgggccaagcccgtgacccagattgtgagcgccgaggcctggggcagggccg
actgcggcttcaccagcgagagctaccagcagggcgtgctgagcgccaccatcctgtacgagatcctgctgggcaaggccaccctgtacgccgtgctggtgtctgccctggtgc
tgatggctatggtgaagcggaaggacagccggggctaa

Fig. 1o

amino acid sequence SEQ ID NO:16
MSIGLLCCAALSLLWAGPVNAGVTQTPKFQVLKTGQSMTLQCAQDMNHEYMSWYRQDPGMGLRLIHYSVGAGTT
DQGEVPNGYNVSRSTTEDFPLRLLSAAPSQTSVYFCASSNVGNTGELFFGEGSRLTVLEDLKNVFPPEVAVFEPSEAE
ISHTQKATLVCLATGFYPDHVELSWWVNGKEVHSGVSTDPQPLKEQPALNDSRYCLSSRLRVSATFWQNPRNHFRC
QVQFYGLSENDEWTQDRAKPVTQIVSAEAWGRADCGFTSESYQQGVLSATILYEILLGKATLYAVLVSALVLMAM
VKRKDSRG.

Fig. 1p

Nucleic acid sequence SEQ ID NO:17
atggagaccctgctgggcctgctgatcctgtggctgcagctccagtgggtgtccagcaagcaggaggtgacccagatccctgccgccctgagcgtgcccgagggcgagaacct
ggtgctgaactgcagcttcaccgactccgccatctacaacctgcagtggttccggcaggaccccggcaagggcctgaccagcctgctgctgattcaatcctcgcagcgggagca
gaccagcggacggctgaacgccagcctggacaagagcagcggccggagcaccctgtacatcgccgccagccagcccggcgacagcgccacctacctgtgcgctgtgcggc
ctatgattggcggcacctacatccccaccttcggcagaggcaccagcctgatcgtgcacccctacatccagaaccccgaccccgccgtgtaccagctgcgggacagcaagagc
agcgacaagtctgtgtgcctgttcaccgacttcgacagccagaccaatgtgagccagagcaaggacagcgacgtgtacatcaccgacaagaccgtgctggacatgcggagcat
ggacttcaagagcaacagcgccgtggcctggagcaacaagagcgacttcgcctgcgccaacgccttcaacaacagcattatccccgaggacaccttcttccccagccccgaga
gcagctgcgacgtgaaactggtggagaagagcttcgagaccgacaccaacctgaacttccagaacctgagcgtgatcggcttcagaatcctgctgctgaaggtggccggattca
acctgctgatgaccctgcggctgtggagcagc

Fig. 1q

amino acid sequence SEQ ID NO:18
METLLGLLILWLQLQWVSSKQEVTQIPAALSVPEGENLVLNCSFTDSAIYNLQWFRQDPGKGLTSLLLIQSSQREQTS
GRLNASLDKSSGRSTLYIAASQPGDSATYLCAVRPMIGGTYIPTFGRGTSLIVHPYIQNPDPAVYQLRDSKSSDKSVC
LFTDFDSQTNVSQSKDSDVYITDKTVLDMRSMDFKSNSAVAWSNKSDFACANAFNNSIIPEDTFFPSPESSCDVKLV
EKSFETDTNLNFQNLSVIGFRILLLKVAGFNLLMTLRLWSS

Fig. 1r

Nucleic acid sequence SEQ ID NO:19
atgagcatcggcctgctgtgctgcgccgccctgagcctgctgtgggcaggacccgtgaacgccggagtgacccagacccccaagttccaggtgctgaaaaccggccagagca
tgaccctgcagtgcgcccaggacatgaaccacgagtacatgagctggtatcggcaggaccccggcatgggcctgcggctgatccactactctgtgggcgcccagaccaccgac
cagggcgaggtgcccaacggctacaatgtgagccggagcaccatcgaggacttccccctgcggctgctgagcgctgcccccagccagaccagcgtgtacttctgcgccagca
gctatctgggcaacaccggcgagctgttcttcggcgagggctccaggctgaccgtgctggaggacctgaagaacgtgttccccccgaggtggccgtgttcgagcccagcgag
gccgagatcagccacacccagaaggccacactggtgtgtctggccaccggcttctaccccgaccacgtggagctgtcctggtgggtgaacggcaaggaggtgcacagcggcg
tgtctaccgacccccagcccctgaaggagcagcccgccctgaacgacagccggtactgcctgtcctccagactgagagtgagcgccaccttctggcagaacccccggaaccac
ttccggtgccaggtgcagttctacggcctgagcgagaacgacgagtggacccaggaccgggccaagcccgtgacccagattgtgagcgccgaggcctggggcagggccgac
tgcggcttcaccagcgagagctaccagcagggcgtgctgagcgccaccatcctgtacgagatcctgctgggcaaggccaccctgtacgccgtgctggtgtctgccctggtgctg
atggctatggtgaagcggaaggacagccggggctaa

Fig. 1s

amino acid sequence SEQ ID NO:20
MSIGLLCCAALSLLWAGPVNAGVTQTPKFQVLKTGQSMTLQCAQDMNHEYMSWYRQDPGMGLRLIHYSVGAQTT
DQGEVPNGYNVSRSTIEDFPLRLLSAAPSQTSVYFCASSYLGNTGELFFGEGSRLTVLEDLKNVFPPEVAVFEPSEAEI
SHTQKATLVCLATGFYPDHVELSWWVNGKEVHSGVSTDPQPLKEQPALNDSRYCLSSRLRVSATFWQNPRNHFRC
QVQFYGLSENDEWTQDRAKPVTQIVSAEAWGRADCGFTSESYQQGVLSATILYEILLGKATLYAVLVSALVLMAM
VKRKDSRG.

Fig. 1t

Nucleic acid sequence SEQ ID NO:21
atggagaccctgctgggcctgctgatcctgtggctgcagctccagtgggtgtccagcaagcaggaggtgacccagatccctgccgccctgagcgtgcccgagggcgagaacct
ggtgctgaactgcagcttcaccgactccgccatctacaacctgcagtggttccggcaggaccccggcaagggcctgaccagcctgctgctgattcaatcctcgcagcgggagca
gaccagcggacggctgaacgccagcctggacaagagcagcggccggagcaccctgtacatcgccgccagccagcccggcgacagcgccacctacctgtgcgctgtgcggc
ctctgctggacggcacctacatccccaccttcggcagaggcaccagcctgatcgtgcacccctacatccagaaccccgaccccgccgtgtaccagctgcgggacagcaagagc
agcgacaagtctgtgtgcctgttcaccgacttcgacagccagaccaatgtgagccagagcaaggacagcgacgtgtacatcaccgacaagaccgtgctggacatgcggagcat
ggacttcaagagcaacagcgccgtggcctggagcaacaagagcgacttcgcctgcgccaacgccttcaacaacagcattatccccgaggacaccttcttccccagccccgaga
gcagctgcgacgtgaaactggtggagaagagcttcgagaccgacaccaacctgaacttccagaacctgagcgtgatcggcttcagaatcctgctgctgaaggtggccggattca
acctgctgatgaccctgcggctgtggagcagc

Fig. 1u

amino acid sequence SEQ ID NO:22
METLLGLLILWLQLQWVSSKQEVTQIPAALSVPEGENLVLNCSFTDSAIYNLQWFRQDPGKGLTSLLLIQSSQREQTS
GRLNASLDKSSGRSTLYIAASQPGDSATYLCAVRPLLDGTYIPTFGRGTSLIVHPYIQNPDPAVYQLRDSKSSDKSVC
LFTDFDSQTNVSQSKDSDVYITDKTVLDMRSMDFKSNSAVAWSNKSDFACANAFNNSIIPEDTFFPSPESSCDVKLV
EKSFETDTNLNFQNLSVIGFRILLLKVAGFNLLMTLRLWSS

Fig. 1v

Nucleic acid sequence SEQ ID NO:23
atgagcatcggcctgctgtgctgcgccgccctgagcctgctgtgggcaggacccgtgaacgccggagtgacccagacccccaagttccaggtgctgaaaaccggccagagca
tgaccctgcagtgcgcccaggacatgaaccacgagtacatgagctggtatcggcaggaccccggcatgggcctgcggctgatccactactctgtgggcgccgggaccaccga
ccgcggcgaggtgcccaacggctacaatgtgagccggagcaccatcgaggacttccccctgcggctgctgagcgctgcccccagccagaccagcgtgtacttctgcgccagc
agctatgtgggcgacaccggcgagctgttcttcggcgagggctccaggctgaccgtgctggaggacctgaagaacgtgttcccccccgaggtggccgtgttcgagcccagcga
ggccgagatcagccacacccagaaggccacactggtgtgtctggccaccggcttctaccccgaccacgtggagctgtcctggtgggtgaacggcaaggaggtgcacagcggc
gtgtctaccgacccccagcccctgaaggagcagcccgccctgaacgacagccggtactgcctgtcctccagactgagagtgagcgccaccttctggcagaacccccggaacc
acttccggtgccaggtgcagttctacggcctgagcgagaacgacgagtggacccaggaccgggccaagcccgtgacccagattgtgagcgccgaggcctggggcagggccg
actgcggcttcaccagcgagagctaccagcagggcgtgctgagcgccaccatcctgtacgagatcctgctgggcaaggccaccctgtacgccgtgctggtgtctgccctggtgc
tgatggctatggtgaagcggaaggacagccggggctaa

Fig. 1w

amino acid sequence SEQ ID NO:24
MSIGLLCCAALSLLWAGPVNAGVTQTPKFQVLKTGQSMTLQCAQDMNHEYMSWYRQDPGMGLRLIHYSVGAGTT
DRGEVPNGYNVSRSTIEDFPLRLLSAAPSQTSVYFCASSYVGDTGELFFGEGSRLTVLEDLKNVFPPEVAVFEPSEAEI
SHTQKATLVCLATGFYPDHVELSWWVNGKEVHSGVSTDPQPLKEQPALNDSRYCLSSRLRVSATFWQNPRNHFRC
QVQFYGLSENDEWTQDRAKPVTQIVSAEAWGRADCGFTSESYQQGVLSATILYEILLGKATLYAVLVSALVLMAM
VKRKDSRG

Fig. 1x

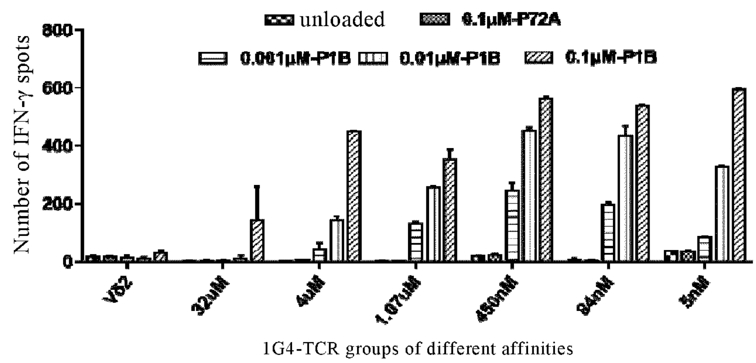


Fig. 2

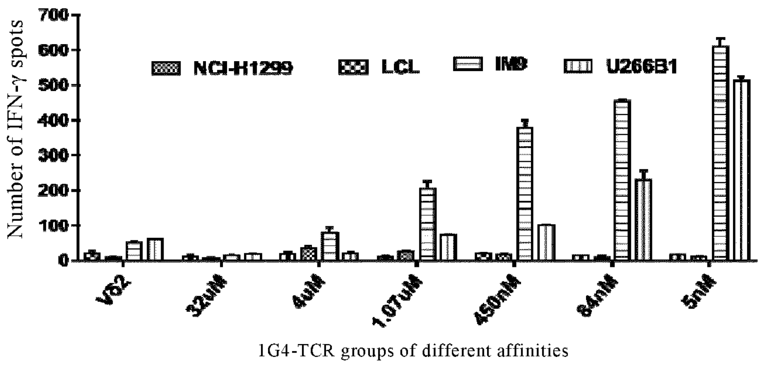


Fig. 3

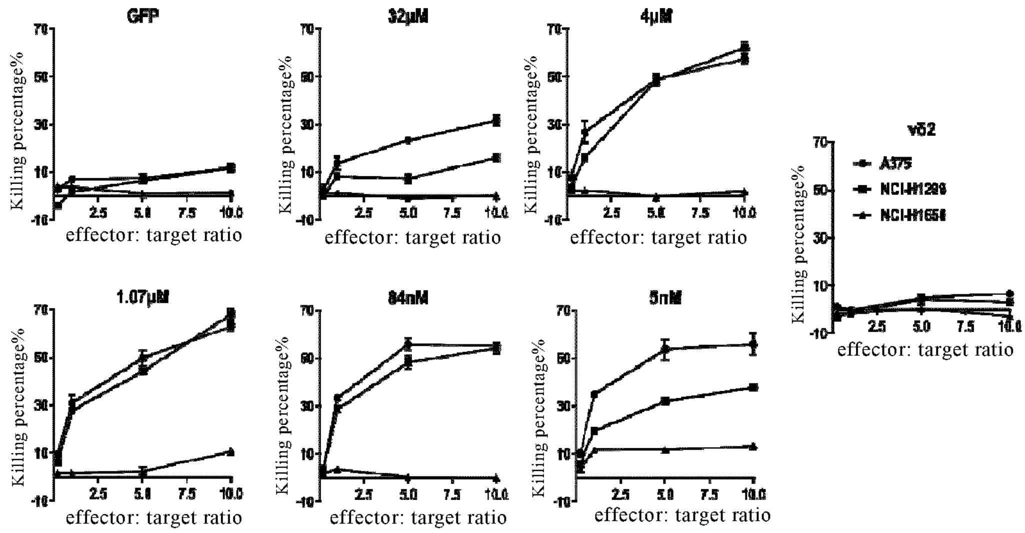


Fig. 4

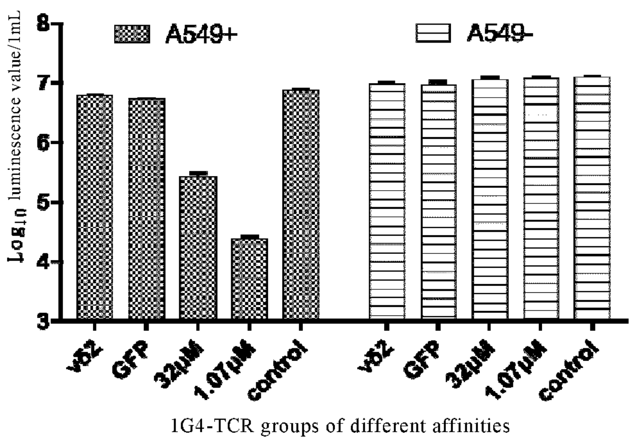


Fig. 5

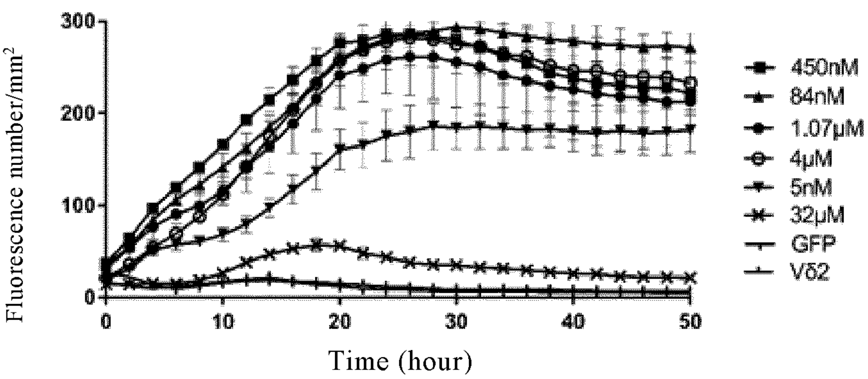


Fig. 6

**INTERNATIONAL SEARCH REPORT**

International application No.
**PCT/CN2018/115235**

**A. CLASSIFICATION OF SUBJECT MATTER**

C12N 5/22(2006.01)i; A61K 39/00(2006.01)i; A61P 31/00(2006.01)i; A61P 35/00(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

数据库: CNABS, CPRSABS, CNMED, TWABS, TWMED, HKABS, MOABS, DWPI, SIPOABS, CNTXT, TWTXT, WOTXT, USTXT, EPTXT, JPTXT, CNKI, WEB OF SCIENCE 关键词: T细胞, T细胞受体, 肿瘤, 感染, ALPHA, BETA, GAMMA, DELTA, T CELL, αβTCR, γδT, αβ, γδ, TUMOR, INFECTION

**C. DOCUMENTS CONSIDERED TO BE RELEVANT**

| Category* | Citation of document, with indication, where appropriate, of the relevant passages | Relevant to claim No. |
|---|---|---|
| X | CN 106075417 A (ZHEJIANG UNIVERSITY) 09 November 2016 (2016-11-09)<br>see abstract, claims 1-4, and description, pp. 9 and 10 | 1-8, 10 |
| A | CN 106795220 A (GENZYME CORP. ET AL.) 31 May 2017 (2017-05-31)<br>see entire document | 1-8, 10 |
| A | HIASA, A. et al. "Rapid αβ TCR-Mediated Responses in γδ T Cells Transduced with Cancer-Specific TCR Genes"<br>*Gene Therapy,* Vol. 16, 31 December 2009 (2009-12-31),<br>ISSN: pp. 0969-7128,<br>pp.620-628, see the entire document | 1-8, 10 |

☐ Further documents are listed in the continuation of Box C. ☑ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **02 January 2019** | **17 January 2019** |
| Name and mailing address of the ISA/CN<br>**State Intellectual Property Office of the P. R. China (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2018/115235

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9**
because they relate to subject matter not required to be searched by this Authority, namely:

[1] A treatment method for the living human or animal body is involved, which falls within the cases set out in PCT Rule 39.1(iv) in which no search is required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2018/115235**

| Patent document cited in search report | | | Publication date (day/month/year) | Patent family member(s) | | | Publication date (day/month/year) |
|---|---|---|---|---|---|---|---|
| CN | 106075417 | A | 09 November 2016 | None | | | |
| CN | 106795220 | A | 31 May 2017 | WO | 2015066379 | A2 | 07 May 2015 |
| | | | | WO | 2015066379 | A3 | 09 July 2015 |
| | | | | MX | 2016005687 | A | 30 March 2017 |
| | | | | AU | 2014342182 | A1 | 19 May 2016 |
| | | | | SG | 10201803473W | A | 28 June 2018 |
| | | | | CA | 2928756 | A1 | 07 May 2015 |
| | | | | US | 2016244523 | A1 | 25 August 2016 |
| | | | | EP | 3063174 | A2 | 07 September 2016 |
| | | | | SG | 11201603193P | A | 30 May 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2016077680 W


### Non-patent literature cited in the description


- **A HIASA1.** *Gene Therapy,* 2009, vol. 16, 620-628
- **JONATHAN M. BOULTER et al.** *Protein Engineering,* 2003, vol. 16 (9), 707-711
- **ROSENBERG et al.** *Nat Rev Cancer,* 2008, vol. 8 (4), 299-308
- **SAMBROOK ; RUSSELL et al.** Molecular cloning: Molecular Cloning-A Laboratory Manual. CSHL, 2001
- **JONATHAN M. BOUTER et al.** *Protein Engineering,* 2003, vol. 16 (9), 707-711
- **YANGBING ZHAO.** *The Journal of Immunology,* 2007, vol. 179, 5845-5854

Sequence listing

<110> GUANGZHOU INSTITUTES OF BIOMEDICINE AND HEALTH, CHINESE ACADEMY OF SCIENCES

<120> GENETICALLY ENGINEERED gamma delte T CELL

<130> P2018-2048

<150> CN2017111240572
<151> 2017-11-14

<160> 24

<170> PatentIn version 3.5

<210> 1
<211> 822
<212> DNA
<213> Artificial sequence

<220>
<223> alpha chain nucleic acid sequence of TCR

<400> 1
atggagaccc tgctgggcct gctgatcctg tggctgcagc tccagtgggt gtccagcaag 60

caggaggtga cccagatccc tgccgccctg agcgtgcccg agggcgagaa cctggtgctg 120

aactgcagct tcaccgactc cgccatctac aacctgcagt ggttccggca ggaccccggc 180

aagggcctga ccagcctgct gctgattcaa tcctcgcagc gggagcagac cagcggacgg 240

ctgaacgcca gcctggacaa gagcagcggc cggagcaccc tgtacatcgc cgccagccag 300

cccggcgaca gcgccaccta cctgtgcgct gtgcggccta cctctggcgg cagctacatc 360

cccaccttcg gcagaggcac cagcctgatc gtgcacccct acatccagaa ccccgacccc 420

gccgtgtacc agctgcggga cagcaagagc agcgacaagt ctgtgtgcct gttcaccgac 480

ttcgacagcc agaccaatgt gagccagagc aaggacagcg acgtgtacat caccgacaag 540

accgtgctgg acatgcggag catggacttc aagagcaaca gcgccgtggc ctggagcaac 600

aagagcgact tcgcctgcgc caacgccttc aacaacagca ttatccccga ggacaccttc 660

ttccccagcc ccgagagcag ctgcgacgtg aaactggtgg agaagagctt cgagaccgac 720

accaacctga acttccagaa cctgagcgtg atcggcttca gaatcctgct gctgaaggtg 780

gccggattca acctgctgat gaccctgcgg ctgtggagca gc 822

<210> 2
<211> 274
<212> PRT
<213> Artificial sequence

<220>
<223> alpha chain amino acid sequence of TCR

<400> 2

```
Met Glu Thr Leu Leu Gly Leu Leu Ile Leu Trp Leu Gln Leu Gln Trp
1               5                   10                  15

Val Ser Ser Lys Gln Glu Val Thr Gln Ile Pro Ala Ala Leu Ser Val
            20                  25                  30

Pro Glu Gly Glu Asn Leu Val Leu Asn Cys Ser Phe Thr Asp Ser Ala
        35                  40                  45

Ile Tyr Asn Leu Gln Trp Phe Arg Gln Asp Pro Gly Lys Gly Leu Thr
    50                  55                  60

Ser Leu Leu Leu Ile Gln Ser Ser Gln Arg Glu Gln Thr Ser Gly Arg
65                  70                  75                  80

Leu Asn Ala Ser Leu Asp Lys Ser Ser Gly Arg Ser Thr Leu Tyr Ile
                85                  90                  95

Ala Ala Ser Gln Pro Gly Asp Ser Ala Thr Tyr Leu Cys Ala Val Arg
            100                 105                 110

Pro Thr Ser Gly Gly Ser Tyr Ile Pro Thr Phe Gly Arg Gly Thr Ser
        115                 120                 125

Leu Ile Val His Pro Tyr Ile Gln Asn Pro Asp Pro Ala Val Tyr Gln
    130                 135                 140

Leu Arg Asp Ser Lys Ser Ser Asp Lys Ser Val Cys Leu Phe Thr Asp
145                 150                 155                 160

Phe Asp Ser Gln Thr Asn Val Ser Gln Ser Lys Asp Ser Asp Val Tyr
                165                 170                 175

Ile Thr Asp Lys Thr Val Leu Asp Met Arg Ser Met Asp Phe Lys Ser
            180                 185                 190

Asn Ser Ala Val Ala Trp Ser Asn Lys Ser Asp Phe Ala Cys Ala Asn
        195                 200                 205

Ala Phe Asn Asn Ser Ile Ile Pro Glu Asp Thr Phe Phe Pro Ser Pro
    210                 215                 220

Glu Ser Ser Cys Asp Val Lys Leu Val Glu Lys Ser Phe Glu Thr Asp
225                 230                 235                 240

Thr Asn Leu Asn Phe Gln Asn Leu Ser Val Ile Gly Phe Arg Ile Leu
                245                 250                 255
```

Leu Leu Lys Val Ala Gly Phe Asn Leu Leu Met Thr Leu Arg Leu Trp
260 265 270

Ser Ser

<210> 3
<211> 936
<212> DNA
<213> Artificial sequence

<220>
<223> beta chain nucleic acid sequence of TCR

<400> 3
atgagcatcg gcctgctgtg ctgcgccgcc ctgagcctgc tgtgggcagg acccgtgaac 60

gccggagtga cccagacccc caagttccag gtgctgaaaa ccggccagag catgaccctg 120

cagtgcgccc aggacatgaa ccacgagtac atgagctggt atcggcagga ccccggcatg 180

ggcctgcggc tgatccacta ctctgtgggc gccgggatca ccgaccaggg cgaggtgccc 240

aacggctaca atgtgagccg gagcaccacc gaggacttcc ccctgcggct gctgagcgct 300

gcccccagcc agaccagcgt gtacttctgc gccagcagct atgtgggcaa caccggcgag 360

ctgttcttcg gcgagggctc caggctgacc gtgctggagg acctgaagaa cgtgttcccc 420

cccgaggtgg ccgtgttcga gcccagcgag gccgagatca gccacaccca gaaggccaca 480

ctggtgtgtc tggccaccgg cttctacccc gaccacgtgg agctgtcctg gtgggtgaac 540

ggcaaggagg tgcacagcgg cgtgtctacc gacccccagc ccctgaagga gcagcccgcc 600

ctgaacgaca gccggtactg cctgtcctcc agactgagag tgagcgccac cttctggcag 660

aacccccgga accacttccg gtgccaggtg cagttctacg gcctgagcga gaacgacgag 720

tggacccagg accgggccaa gcccgtgacc cagattgtga gcgccgaggc ctggggcagg 780

gccgactgcg gcttcaccag cgagagctac cagcagggcg tgctgagcgc caccatcctg 840

tacgagatcc tgctgggcaa ggccaccctg tacgccgtgc tggtgtctgc cctggtgctg 900

atggctatgg tgaagcggaa ggacagccgg ggctaa 936

<210> 4
<211> 311
<212> PRT
<213> Artificial sequence

<220>
<223> beta chain amino acid sequence of TCR

<400> 4

Met Ser Ile Gly Leu Leu Cys Cys Ala Ala Leu Ser Leu Leu Trp Ala

1 5 10 15

Gly Pro Val Asn Ala Gly Val Thr Gln Thr Pro Lys Phe Gln Val Leu
20 25 30

Lys Thr Gly Gln Ser Met Thr Leu Gln Cys Ala Gln Asp Met Asn His
35 40 45

Glu Tyr Met Ser Trp Tyr Arg Gln Asp Pro Gly Met Gly Leu Arg Leu
50 55 60

Ile His Tyr Ser Val Gly Ala Gly Ile Thr Asp Gln Gly Glu Val Pro
65 70 75 80

Asn Gly Tyr Asn Val Ser Arg Ser Thr Thr Glu Asp Phe Pro Leu Arg
85 90 95

Leu Leu Ser Ala Ala Pro Ser Gln Thr Ser Val Tyr Phe Cys Ala Ser
100 105 110

Ser Tyr Val Gly Asn Thr Gly Glu Leu Phe Phe Gly Glu Gly Ser Arg
115 120 125

Leu Thr Val Leu Glu Asp Leu Lys Asn Val Phe Pro Pro Glu Val Ala
130 135 140

Val Phe Glu Pro Ser Glu Ala Glu Ile Ser His Thr Gln Lys Ala Thr
145 150 155 160

Leu Val Cys Leu Ala Thr Gly Phe Tyr Pro Asp His Val Glu Leu Ser
165 170 175

Trp Trp Val Asn Gly Lys Glu Val His Ser Gly Val Ser Thr Asp Pro
180 185 190

Gln Pro Leu Lys Glu Gln Pro Ala Leu Asn Asp Ser Arg Tyr Cys Leu
195 200 205

Ser Ser Arg Leu Arg Val Ser Ala Thr Phe Trp Gln Asn Pro Arg Asn
210 215 220

His Phe Arg Cys Gln Val Gln Phe Tyr Gly Leu Ser Glu Asn Asp Glu
225 230 235 240

Trp Thr Gln Asp Arg Ala Lys Pro Val Thr Gln Ile Val Ser Ala Glu
245 250 255

Ala Trp Gly Arg Ala Asp Cys Gly Phe Thr Ser Glu Ser Tyr Gln Gln
260 265 270

Gly Val Leu Ser Ala Thr Ile Leu Tyr Glu Ile Leu Leu Gly Lys Ala
275 280 285

Thr Leu Tyr Ala Val Leu Val Ser Ala Leu Val Leu Met Ala Met Val
290 295 300

Lys Arg Lys Asp Ser Arg Gly
305 310

<210> 5
<211> 822
<212> DNA
<213> Artificial sequence

<220>
<223> alpha chain nucleic acid sequence of TCR

<400> 5
atggagaccc tgctgggcct gctgatcctg tggctgcagc tccagtgggt gtccagcaag 60

caggaggtga cccagatccc tgccgccctg agcgtgcccg agggcgagaa cctggtgctg 120

aactgcagct tcaccgactc cgccatctac aacctgcagt ggttccggca ggaccccggc 180

aagggcctga ccagcctgct gctgattcaa tcctcgcagc gggagcagac cagcggacgg 240

ctgaacgcca gcctggacaa gagcagcggc cggagcaccc tgtacatcgc cgccagccag 300

cccggcgaca gcgccaccta cctgtgcgct gtgcggccta cctctggcgg cagctacatc 360

cccaccttcg gcagaggcac cagcctgatc gtgcacccct acatccagaa ccccgacccc 420

gccgtgtacc agctgcggga cagcaagagc agcgacaagt ctgtgtgcct gttcaccgac 480

ttcgacagcc agaccaatgt gagccagagc aaggacagcg acgtgtacat caccgacaag 540

accgtgctgg acatgcggag catggacttc aagagcaaca gcgccgtggc ctggagcaac 600

aagagcgact tcgcctgcgc caacgccttc aacaacagca ttatccccga ggacaccttc 660

ttccccagcc ccgagagcag ctgcgacgtg aaactggtgg agaagagctt cgagaccgac 720

accaacctga acttccagaa cctgagcgtg atcggcttca gaatcctgct gctgaaggtg 780

gccggattca acctgctgat gaccctgcgg ctgtggagca gc 822

<210> 6
<211> 274
<212> PRT
<213> Artificial sequence

<220>
<223> alpha chain amino acid sequence of TCR

<400> 6

```
Met Glu Thr Leu Leu Gly Leu Leu Ile Leu Trp Leu Gln Leu Gln Trp
1               5                   10                  15

Val Ser Ser Lys Gln Glu Val Thr Gln Ile Pro Ala Ala Leu Ser Val
            20                  25                  30

Pro Glu Gly Glu Asn Leu Val Leu Asn Cys Ser Phe Thr Asp Ser Ala
        35                  40                  45

Ile Tyr Asn Leu Gln Trp Phe Arg Gln Asp Pro Gly Lys Gly Leu Thr
    50                  55                  60

Ser Leu Leu Leu Ile Gln Ser Ser Gln Arg Glu Gln Thr Ser Gly Arg
65                  70                  75                  80

Leu Asn Ala Ser Leu Asp Lys Ser Ser Gly Arg Ser Thr Leu Tyr Ile
                85                  90                  95

Ala Ala Ser Gln Pro Gly Asp Ser Ala Thr Tyr Leu Cys Ala Val Arg
            100                 105                 110

Pro Thr Ser Gly Gly Ser Tyr Ile Pro Thr Phe Gly Arg Gly Thr Ser
        115                 120                 125

Leu Ile Val His Pro Tyr Ile Gln Asn Pro Asp Pro Ala Val Tyr Gln
    130                 135                 140

Leu Arg Asp Ser Lys Ser Ser Asp Lys Ser Val Cys Leu Phe Thr Asp
145                 150                 155                 160

Phe Asp Ser Gln Thr Asn Val Ser Gln Ser Lys Asp Ser Asp Val Tyr
                165                 170                 175

Ile Thr Asp Lys Thr Val Leu Asp Met Arg Ser Met Asp Phe Lys Ser
            180                 185                 190

Asn Ser Ala Val Ala Trp Ser Asn Lys Ser Asp Phe Ala Cys Ala Asn
        195                 200                 205

Ala Phe Asn Asn Ser Ile Ile Pro Glu Asp Thr Phe Phe Pro Ser Pro
    210                 215                 220

Glu Ser Ser Cys Asp Val Lys Leu Val Glu Lys Ser Phe Glu Thr Asp
225                 230                 235                 240

Thr Asn Leu Asn Phe Gln Asn Leu Ser Val Ile Gly Phe Arg Ile Leu
                245                 250                 255
```

Leu Leu Lys Val Ala Gly Phe Asn Leu Leu Met Thr Leu Arg Leu Trp
260 265 270

Ser Ser

<210> 7
<211> 936
<212> DNA
<213> Artificial sequence

<220>
<223> beta chain nucleic acid sequence of TCR

<400> 7
atgagcatcg gcctgctgtg ctgcgccgcc ctgagcctgc tgtgggcagg acccgtgaac 60

gccggagtga cccagacccc caagttccag gtgctgaaaa ccggccagag catgaccctg 120

cagtgcgccc aggacatgaa ccacgagtac atgagctggt atcggcagga ccccggcatg 180

ggcctgcggc tgatccacta ctctgtgggc gccgggacca ccgaccaggg cgaggtgccc 240

aacggctaca atgtgagccg gagcaccacc gaggacttcc ccctgcggct gctgagcgct 300

gcccccagcc agaccagcgt gtacttctgc gccagcagct atctgggcga caccggcgag 360

ctgttcttcg gcgagggctc caggctgacc gtgctggagg acctgaagaa cgtgttcccc 420

cccgaggtgg ccgtgttcga gcccagcgag gccgagatca gccacaccca gaaggccaca 480

ctggtgtgtc tggccaccgg cttctacccc gaccacgtgg agctgtcctg gtgggtgaac 540

ggcaaggagg tgcacagcgg cgtgtctacc gacccccagc ccctgaagga gcagcccgcc 600

ctgaacgaca gccggtactg cctgtcctcc agactgagag tgagcgccac cttctggcag 660

aacccccgga accacttccg gtgccaggtg cagttctacg gcctgagcga gaacgacgag 720

tggacccagg accgggccaa gcccgtgacc cagattgtga gcgccgaggc ctggggcagg 780

gccgactgcg gcttcaccag cgagagctac cagcagggcg tgctgagcgc caccatcctg 840

tacgagatcc tgctgggcaa ggccaccctg tacgccgtgc tggtgtctgc cctggtgctg 900

atggctatgg tgaagcggaa ggacagccgg ggctaa 936

<210> 8
<211> 311
<212> PRT
<213> Artificial sequence

<220>
<223> beta chain amino acid sequence of TCR

<400> 8

Met Ser Ile Gly Leu Leu Cys Cys Ala Ala Leu Ser Leu Leu Trp Ala

```
1               5                   10                  15

Gly Pro Val Asn Ala Gly Val Thr Gln Thr Pro Lys Phe Gln Val Leu
            20                  25                  30

Lys Thr Gly Gln Ser Met Thr Leu Gln Cys Ala Gln Asp Met Asn His
        35                  40                  45

Glu Tyr Met Ser Trp Tyr Arg Gln Asp Pro Gly Met Gly Leu Arg Leu
    50                  55                  60

Ile His Tyr Ser Val Gly Ala Gly Thr Thr Asp Gln Gly Glu Val Pro
65                  70                  75                  80

Asn Gly Tyr Asn Val Ser Arg Ser Thr Thr Glu Asp Phe Pro Leu Arg
                85                  90                  95

Leu Leu Ser Ala Ala Pro Ser Gln Thr Ser Val Tyr Phe Cys Ala Ser
            100                 105                 110

Ser Tyr Leu Gly Asp Thr Gly Glu Leu Phe Phe Gly Glu Gly Ser Arg
        115                 120                 125

Leu Thr Val Leu Glu Asp Leu Lys Asn Val Phe Pro Pro Glu Val Ala
    130                 135                 140

Val Phe Glu Pro Ser Glu Ala Glu Ile Ser His Thr Gln Lys Ala Thr
145                 150                 155                 160

Leu Val Cys Leu Ala Thr Gly Phe Tyr Pro Asp His Val Glu Leu Ser
                165                 170                 175

Trp Trp Val Asn Gly Lys Glu Val His Ser Gly Val Ser Thr Asp Pro
            180                 185                 190

Gln Pro Leu Lys Glu Gln Pro Ala Leu Asn Asp Ser Arg Tyr Cys Leu
        195                 200                 205

Ser Ser Arg Leu Arg Val Ser Ala Thr Phe Trp Gln Asn Pro Arg Asn
    210                 215                 220

His Phe Arg Cys Gln Val Gln Phe Tyr Gly Leu Ser Glu Asn Asp Glu
225                 230                 235                 240

Trp Thr Gln Asp Arg Ala Lys Pro Val Thr Gln Ile Val Ser Ala Glu
                245                 250                 255
```

Ala Trp Gly Arg Ala Asp Cys Gly Phe Thr Ser Glu Ser Tyr Gln Gln
260 265 270

Gly Val Leu Ser Ala Thr Ile Leu Tyr Glu Ile Leu Leu Gly Lys Ala
275 280 285

Thr Leu Tyr Ala Val Leu Val Ser Ala Leu Val Leu Met Ala Met Val
290 295 300

Lys Arg Lys Asp Ser Arg Gly
305 310

<210> 9
<211> 822
<212> DNA
<213> Artificial sequence

<220>
<223> alpha chain nucleic acid sequence of TCR

<400> 9
atggagaccc tgctgggcct gctgatcctg tggctgcagc tgcagtgggt gagctccaag 60

caggaggtga cacagatccc tgccgccctg agcgtgccag agggagagaa cctggtgctg 120

aattgctctt ttaccgacag cgccatctac aacctgcagt ggttccggca ggatccaggc 180

aagggcctga cctctctgct gctgatccag tctagccagc gggagcagac atctggcaga 240

ctgaatgcca gcctggacaa gtcctctggc agatccaccc tgtacatcgc agcctcccag 300

ccaggcgatt ctgccacata tctgtgcgcc gtgaggccac tgtacggagg aagctatatc 360

cctacctttg gccgcggcac atccctgatc gtgcaccctt acatccagaa cccagacccc 420

gccgtgtatc agctgaggga ctccaagagc tccgataaga gcgtgtgcct gttcaccgac 480

tttgattctc agacaaacgt gagccagtct aaggacagcg acgtgtacat caccgacaag 540

acagtgctgg atatgcgctc catggacttc aagagcaact ccgccgtggc ctggtctaat 600

aagagcgatt tcgcctgcgc caacgccttt aacaattcca tcatccctga ggatacctc 660

tttccttctc cagagtctag ctgtgacgtg aagctggtgg agaagtcctt cgagaccgat 720

acaaacctga attttcagaa cctgtctgtg atcggcttca ggatcctgct gctgaaggtg 780

gccggcttta atctgctgat gaccctgagg ctgtggtcct ct 822

<210> 10
<211> 274
<212> PRT
<213> Artificial sequence

<220>
<223> alpha chain amino acid sequence of TCR

<400> 10

```
Met Glu Thr Leu Leu Gly Leu Leu Ile Leu Trp Leu Gln Leu Gln Trp
1               5                   10                  15

Val Ser Ser Lys Gln Glu Val Thr Gln Ile Pro Ala Ala Leu Ser Val
            20                  25                  30

Pro Glu Gly Glu Asn Leu Val Leu Asn Cys Ser Phe Thr Asp Ser Ala
        35                  40                  45

Ile Tyr Asn Leu Gln Trp Phe Arg Gln Asp Pro Gly Lys Gly Leu Thr
    50                  55                  60

Ser Leu Leu Leu Ile Gln Ser Ser Gln Arg Glu Gln Thr Ser Gly Arg
65                  70                  75                  80

Leu Asn Ala Ser Leu Asp Lys Ser Ser Gly Arg Ser Thr Leu Tyr Ile
                85                  90                  95

Ala Ala Ser Gln Pro Gly Asp Ser Ala Thr Tyr Leu Cys Ala Val Arg
            100                 105                 110

Pro Leu Tyr Gly Gly Ser Tyr Ile Pro Thr Phe Gly Arg Gly Thr Ser
        115                 120                 125

Leu Ile Val His Pro Tyr Ile Gln Asn Pro Asp Pro Ala Val Tyr Gln
    130                 135                 140

Leu Arg Asp Ser Lys Ser Ser Asp Lys Ser Val Cys Leu Phe Thr Asp
145                 150                 155                 160

Phe Asp Ser Gln Thr Asn Val Ser Gln Ser Lys Asp Ser Asp Val Tyr
                165                 170                 175

Ile Thr Asp Lys Thr Val Leu Asp Met Arg Ser Met Asp Phe Lys Ser
            180                 185                 190

Asn Ser Ala Val Ala Trp Ser Asn Lys Ser Asp Phe Ala Cys Ala Asn
        195                 200                 205

Ala Phe Asn Asn Ser Ile Ile Pro Glu Asp Thr Phe Phe Pro Ser Pro
    210                 215                 220

Glu Ser Ser Cys Asp Val Lys Leu Val Glu Lys Ser Phe Glu Thr Asp
225                 230                 235                 240

Thr Asn Leu Asn Phe Gln Asn Leu Ser Val Ile Gly Phe Arg Ile Leu
                245                 250                 255
```

Leu Leu Lys Val Ala Gly Phe Asn Leu Leu Met Thr Leu Arg Leu Trp
260 265 270

Ser Ser

<210> 11
<211> 936
<212> DNA
<213> Artificial sequence

<220>
<223> beta chain nucleic acid sequence of TCR

<400> 11
atgagcatcg gactgctgtg ctgtgccgcc ctgtccctgc tgtgggcagg acctgtgaac 60

gcaggagtga cccagacacc aaagtttcag gtgctgaaga ccggccagtc catgacactg 120

cagtgcgccc aggacatgaa tcacgagtac atgtcttggt ataggcagga tccaggaatg 180

ggactgaggc tgatccacta cagcgtggga gcaggaatca cagaccaggg agaggtgcct 240

aacggctata acgtgagccg gtctaccaca gaggattttc cactgagact gctgagcgcc 300

gcaccatccc agaccagcgt gtacttctgc gccagctcct atgtgggcaa caccggcgag 360

ctgttctttg gagagggatc ccggctgaca gtgctggagg acctgaagaa cgtgttcccc 420

cctgaggtgg ccgtgtttga gccaagcgag gccgagatct cccacaccca gaaggccacc 480

ctggtgtgcc tggccacagg cttctacccc gatcacgtgg agctgagctg gtgggtgaac 540

ggcaaggagg tgcacagcgg cgtgtccacc gacccacagc ccctgaagga gcagcctgcc 600

ctgaatgatt ctagatactg cctgtctagc cggctgagag tgagcgccac cttttggcag 660

aacccacgga atcacttcag atgtcaggtg cagttttatg gcctgagcga gaacgatgag 720

tggacccagg acagggcaaa gccagtgaca cagatcgtgt ccgccgaggc atggggaaga 780

gcagactgtg gcttcaccag cgagtcctat cagcagggcg tgctgtccgc caccatcctg 840

tacgagatcc tgctgggcaa ggccacactg tatgccgtgc tggtgtctgc cctggtgctg 900

atggccatgg tgaagaggaa ggatagccgc ggctaa 936

<210> 12
<211> 311
<212> PRT
<213> Artificial sequence

<220>
<223> beta chain amino acid sequence of TCR

<400> 12

Met Ser Ile Gly Leu Leu Cys Cys Ala Ala Leu Ser Leu Leu Trp Ala

1 5 10 15

Gly Pro Val Asn Ala Gly Val Thr Gln Thr Pro Lys Phe Gln Val Leu
20 25 30

Lys Thr Gly Gln Ser Met Thr Leu Gln Cys Ala Gln Asp Met Asn His
35 40 45

Glu Tyr Met Ser Trp Tyr Arg Gln Asp Pro Gly Met Gly Leu Arg Leu
50 55 60

Ile His Tyr Ser Val Gly Ala Gly Ile Thr Asp Gln Gly Glu Val Pro
65 70 75 80

Asn Gly Tyr Asn Val Ser Arg Ser Thr Thr Glu Asp Phe Pro Leu Arg
85 90 95

Leu Leu Ser Ala Ala Pro Ser Gln Thr Ser Val Tyr Phe Cys Ala Ser
100 105 110

Ser Tyr Val Gly Asn Thr Gly Glu Leu Phe Phe Gly Glu Gly Ser Arg
115 120 125

Leu Thr Val Leu Glu Asp Leu Lys Asn Val Phe Pro Pro Glu Val Ala
130 135 140

Val Phe Glu Pro Ser Glu Ala Glu Ile Ser His Thr Gln Lys Ala Thr
145 150 155 160

Leu Val Cys Leu Ala Thr Gly Phe Tyr Pro Asp His Val Glu Leu Ser
165 170 175

Trp Trp Val Asn Gly Lys Glu Val His Ser Gly Val Ser Thr Asp Pro
180 185 190

Gln Pro Leu Lys Glu Gln Pro Ala Leu Asn Asp Ser Arg Tyr Cys Leu
195 200 205

Ser Ser Arg Leu Arg Val Ser Ala Thr Phe Trp Gln Asn Pro Arg Asn
210 215 220

His Phe Arg Cys Gln Val Gln Phe Tyr Gly Leu Ser Glu Asn Asp Glu
225 230 235 240

Trp Thr Gln Asp Arg Ala Lys Pro Val Thr Gln Ile Val Ser Ala Glu
245 250 255

Ala Trp Gly Arg Ala Asp Cys Gly Phe Thr Ser Glu Ser Tyr Gln Gln
260 265 270

Gly Val Leu Ser Ala Thr Ile Leu Tyr Glu Ile Leu Leu Gly Lys Ala
275 280 285

Thr Leu Tyr Ala Val Leu Val Ser Ala Leu Val Leu Met Ala Met Val
290 295 300

Lys Arg Lys Asp Ser Arg Gly
305 310

<210> 13
<211> 822
<212> DNA
<213> Artificial sequence

<220>
<223> alpha chain nucleic acid sequence of TCR

<400> 13
atggagaccc tgctgggcct gctgatcctg tggctgcagc tccagtgggt gtccagcaag 60

caggaggtga cccagatccc tgccgccctg agcgtgcccg agggcgagaa cctggtgctg 120

aactgcagct tcaccgactc cgccatctac aacctgcagt ggttccggca ggaccccggc 180

aagggcctga ccagcctgct gctgattcaa tcctcgcagc gggagcagac cagcggacgg 240

ctgaacgcca gcctggacaa gagcagcggc cggagcaccc tgtacatcgc cgccagccag 300

cccggcgaca gcgccaccta cctgtgcgct gtgcggcctc tgacaggcgg cagctacatc 360

cccaccttcg gcagaggcac cagcctgatc gtgcacccct acatccagaa ccccgacccc 420

gccgtgtacc agctgcggga cagcaagagc agcgacaagt ctgtgtgcct gttcaccgac 480

ttcgacagcc agaccaatgt gagccagagc aaggacagcg acgtgtacat caccgacaag 540

accgtgctgg acatgcggag catggacttc aagagcaaca gcgccgtggc ctggagcaac 600

aagagcgact tcgcctgcgc caacgccttc aacaacagca ttatccccga ggacaccttc 660

ttccccagcc ccgagagcag ctgcgacgtg aaactggtgg agaagagctt cgagaccgac 720

accaacctga acttccagaa cctgagcgtg atcggcttca gaatcctgct gctgaaggtg 780

gccggattca acctgctgat gaccctgcgg ctgtggagca gc 822

<210> 14
<211> 274
<212> PRT
<213> Artificial sequence

<220>
<223> alpha chain amino acid sequence of TCR

<400> 14

Met Glu Thr Leu Leu Gly Leu Leu Ile Leu Trp Leu Gln Leu Gln Trp
1               5                   10                  15

Val Ser Ser Lys Gln Glu Val Thr Gln Ile Pro Ala Ala Leu Ser Val
            20                  25                  30

Pro Glu Gly Glu Asn Leu Val Leu Asn Cys Ser Phe Thr Asp Ser Ala
        35                  40                  45

Ile Tyr Asn Leu Gln Trp Phe Arg Gln Asp Pro Gly Lys Gly Leu Thr
    50                  55                  60

Ser Leu Leu Leu Ile Gln Ser Ser Gln Arg Glu Gln Thr Ser Gly Arg
65                  70                  75                  80

Leu Asn Ala Ser Leu Asp Lys Ser Ser Gly Arg Ser Thr Leu Tyr Ile
                85                  90                  95

Ala Ala Ser Gln Pro Gly Asp Ser Ala Thr Tyr Leu Cys Ala Val Arg
            100                 105                 110

Pro Leu Thr Gly Gly Ser Tyr Ile Pro Thr Phe Gly Arg Gly Thr Ser
        115                 120                 125

Leu Ile Val His Pro Tyr Ile Gln Asn Pro Asp Pro Ala Val Tyr Gln
    130                 135                 140

Leu Arg Asp Ser Lys Ser Ser Asp Lys Ser Val Cys Leu Phe Thr Asp
145                 150                 155                 160

Phe Asp Ser Gln Thr Asn Val Ser Gln Ser Lys Asp Ser Asp Val Tyr
                165                 170                 175

Ile Thr Asp Lys Thr Val Leu Asp Met Arg Ser Met Asp Phe Lys Ser
            180                 185                 190

Asn Ser Ala Val Ala Trp Ser Asn Lys Ser Asp Phe Ala Cys Ala Asn
        195                 200                 205

Ala Phe Asn Asn Ser Ile Ile Pro Glu Asp Thr Phe Phe Pro Ser Pro
    210                 215                 220

Glu Ser Ser Cys Asp Val Lys Leu Val Glu Lys Ser Phe Glu Thr Asp
225                 230                 235                 240

Thr Asn Leu Asn Phe Gln Asn Leu Ser Val Ile Gly Phe Arg Ile Leu
                245                 250                 255

Leu Leu Lys Val Ala Gly Phe Asn Leu Leu Met Thr Leu Arg Leu Trp
260 265 270

Ser Ser

<210> 15
<211> 936
<212> DNA
<213> Artificial sequence

<220>
<223> beta chain nucleic acid sequence of TCR

<400> 15
atgagcatcg gcctgctgtg ctgcgccgcc ctgagcctgc tgtgggcagg acccgtgaac 60

gccggagtga cccagacccc caagttccag gtgctgaaaa ccggccagag catgaccctg 120

cagtgcgccc aggacatgaa ccacgagtac atgagctggt atcggcagga ccccggcatg 180

ggcctgcggc tgatccacta ctctgtgggc gccgggacca ccgaccaggg cgaggtgccc 240

aacggctaca atgtgagccg gagcaccacc gaggacttcc ccctgcggct gctgagcgct 300

gcccccagcc agaccagcgt gtacttctgc gccagcagca atgtgggcaa caccggcgag 360

ctgttcttcg gcgagggctc caggctgacc gtgctggagg acctgaagaa cgtgttcccc 420

cccgaggtgg ccgtgttcga gcccagcgag gccgagatca gccacaccca gaaggccaca 480

ctggtgtgtc tggccaccgg cttctacccc gaccacgtgg agctgtcctg gtgggtgaac 540

ggcaaggagg tgcacagcgg cgtgtctacc gacccccagc ccctgaagga gcagcccgcc 600

ctgaacgaca gccggtactg cctgtcctcc agactgagag tgagcgccac cttctggcag 660

aacccccgga accacttccg gtgccaggtg cagttctacg gcctgagcga gaacgacgag 720

tggacccagg accgggccaa gcccgtgacc cagattgtga gcgccgaggc ctggggcagg 780

gccgactgcg gcttcaccag cgagagctac cagcagggcg tgctgagcgc caccatcctg 840

tacgagatcc tgctgggcaa ggccaccctg tacgccgtgc tggtgtctgc cctggtgctg 900

atggctatgg tgaagcggaa ggacagccgg ggctaa 936

<210> 16
<211> 311
<212> PRT
<213> Artificial sequence

<220>
<223> beta chain amino acid sequence of TCR

<400> 16

Met Ser Ile Gly Leu Leu Cys Cys Ala Ala Leu Ser Leu Leu Trp Ala

```
1               5                   10                  15

Gly Pro Val Asn Ala Gly Val Thr Gln Thr Pro Lys Phe Gln Val Leu
            20                  25                  30

Lys Thr Gly Gln Ser Met Thr Leu Gln Cys Ala Gln Asp Met Asn His
        35                  40                  45

Glu Tyr Met Ser Trp Tyr Arg Gln Asp Pro Gly Met Gly Leu Arg Leu
    50                  55                  60

Ile His Tyr Ser Val Gly Ala Gly Thr Thr Asp Gln Gly Glu Val Pro
65                  70                  75                  80

Asn Gly Tyr Asn Val Ser Arg Ser Thr Thr Glu Asp Phe Pro Leu Arg
                85                  90                  95

Leu Leu Ser Ala Ala Pro Ser Gln Thr Ser Val Tyr Phe Cys Ala Ser
            100                 105                 110

Ser Asn Val Gly Asn Thr Gly Glu Leu Phe Phe Gly Glu Gly Ser Arg
        115                 120                 125

Leu Thr Val Leu Glu Asp Leu Lys Asn Val Phe Pro Pro Glu Val Ala
    130                 135                 140

Val Phe Glu Pro Ser Glu Ala Glu Ile Ser His Thr Gln Lys Ala Thr
145                 150                 155                 160

Leu Val Cys Leu Ala Thr Gly Phe Tyr Pro Asp His Val Glu Leu Ser
                165                 170                 175

Trp Trp Val Asn Gly Lys Glu Val His Ser Gly Val Ser Thr Asp Pro
            180                 185                 190

Gln Pro Leu Lys Glu Gln Pro Ala Leu Asn Asp Ser Arg Tyr Cys Leu
        195                 200                 205

Ser Ser Arg Leu Arg Val Ser Ala Thr Phe Trp Gln Asn Pro Arg Asn
    210                 215                 220

His Phe Arg Cys Gln Val Gln Phe Tyr Gly Leu Ser Glu Asn Asp Glu
225                 230                 235                 240

Trp Thr Gln Asp Arg Ala Lys Pro Val Thr Gln Ile Val Ser Ala Glu
                245                 250                 255
```

```
Ala Trp Gly Arg Ala Asp Cys Gly Phe Thr Ser Glu Ser Tyr Gln Gln
            260                 265                 270

Gly Val Leu Ser Ala Thr Ile Leu Tyr Glu Ile Leu Leu Gly Lys Ala
        275                 280                 285

Thr Leu Tyr Ala Val Leu Val Ser Ala Leu Val Leu Met Ala Met Val
    290                 295                 300

Lys Arg Lys Asp Ser Arg Gly
305                 310


<210>  17
<211>  822
<212>  DNA
<213>  Artificial sequence

<220>
<223>  alpha chain nucleic acid sequence of TCR

<400>  17
atggagaccc tgctgggcct gctgatcctg tggctgcagc tccagtgggt gtccagcaag       60

caggaggtga cccagatccc tgccgccctg agcgtgcccg agggcgagaa cctggtgctg      120

aactgcagct tcaccgactc cgccatctac aacctgcagt ggttccggca ggaccccggc      180

aagggcctga ccagcctgct gctgattcaa tcctcgcagc gggagcagac cagcggacgg      240

ctgaacgcca gcctggacaa gagcagcggc cggagcaccc tgtacatcgc cgccagccag      300

cccggcgaca gcgccaccta cctgtgcgct gtgcggccta tgattggcgg cacctacatc      360

cccaccttcg gcagaggcac cagcctgatc gtgcacccct acatccagaa ccccgacccc      420

gccgtgtacc agctgcggga cagcaagagc agcgacaagt ctgtgtgcct gttcaccgac      480

ttcgacagcc agaccaatgt gagccagagc aaggacagcg acgtgtacat caccgacaag      540

accgtgctgg acatgcggag catggacttc aagagcaaca gcgccgtggc ctggagcaac      600

aagagcgact tcgcctgcgc caacgccttc aacaacagca ttatccccga ggacaccttc      660

ttccccagcc ccgagagcag ctgcgacgtg aaactggtgg agaagagctt cgagaccgac      720

accaacctga acttccagaa cctgagcgtg atcggcttca gaatcctgct gctgaaggtg      780

gccggattca acctgctgat gaccctgcgg ctgtggagca gc                         822


<210>  18
<211>  274
<212>  PRT
<213>  Artificial sequence

<220>
<223>  alpha chain amino acid sequence of TCR

<400>  18
```

```
Met Glu Thr Leu Leu Gly Leu Leu Ile Leu Trp Leu Gln Leu Gln Trp
1               5                   10                  15

Val Ser Ser Lys Gln Glu Val Thr Gln Ile Pro Ala Ala Leu Ser Val
            20                  25                  30

Pro Glu Gly Glu Asn Leu Val Leu Asn Cys Ser Phe Thr Asp Ser Ala
        35                  40                  45

Ile Tyr Asn Leu Gln Trp Phe Arg Gln Asp Pro Gly Lys Gly Leu Thr
    50                  55                  60

Ser Leu Leu Leu Ile Gln Ser Ser Gln Arg Glu Gln Thr Ser Gly Arg
65                  70                  75                  80

Leu Asn Ala Ser Leu Asp Lys Ser Ser Gly Arg Ser Thr Leu Tyr Ile
                85                  90                  95

Ala Ala Ser Gln Pro Gly Asp Ser Ala Thr Tyr Leu Cys Ala Val Arg
            100                 105                 110

Pro Met Ile Gly Gly Thr Tyr Ile Pro Thr Phe Gly Arg Gly Thr Ser
        115                 120                 125

Leu Ile Val His Pro Tyr Ile Gln Asn Pro Asp Pro Ala Val Tyr Gln
    130                 135                 140

Leu Arg Asp Ser Lys Ser Ser Asp Lys Ser Val Cys Leu Phe Thr Asp
145                 150                 155                 160

Phe Asp Ser Gln Thr Asn Val Ser Gln Ser Lys Asp Ser Asp Val Tyr
                165                 170                 175

Ile Thr Asp Lys Thr Val Leu Asp Met Arg Ser Met Asp Phe Lys Ser
            180                 185                 190

Asn Ser Ala Val Ala Trp Ser Asn Lys Ser Asp Phe Ala Cys Ala Asn
        195                 200                 205

Ala Phe Asn Asn Ser Ile Ile Pro Glu Asp Thr Phe Phe Pro Ser Pro
    210                 215                 220

Glu Ser Ser Cys Asp Val Lys Leu Val Glu Lys Ser Phe Glu Thr Asp
225                 230                 235                 240

Thr Asn Leu Asn Phe Gln Asn Leu Ser Val Ile Gly Phe Arg Ile Leu
                245                 250                 255
```

Leu Leu Lys Val Ala Gly Phe Asn Leu Leu Met Thr Leu Arg Leu Trp
260 265 270

Ser Ser

<210> 19
<211> 936
<212> DNA
<213> Artificial sequence

<220>
<223> beta chain nucleic acid sequence of TCR

<400> 19
atgagcatcg gcctgctgtg ctgcgccgcc ctgagcctgc tgtgggcagg acccgtgaac 60

gccggagtga cccagacccc caagttccag gtgctgaaaa ccggccagag catgaccctg 120

cagtgcgccc aggacatgaa ccacgagtac atgagctggt atcggcagga ccccggcatg 180

ggcctgcggc tgatccacta ctctgtgggc gcccagacca ccgaccaggg cgaggtgccc 240

aacggctaca atgtgagccg gagcaccatc gaggacttcc ccctgcggct gctgagcgct 300

gcccccagcc agaccagcgt gtacttctgc gccagcagct atctgggcaa caccggcgag 360

ctgttcttcg gcgagggctc caggctgacc gtgctggagg acctgaagaa cgtgttcccc 420

cccgaggtgg ccgtgttcga gcccagcgag gccgagatca gccacaccca gaaggccaca 480

ctggtgtgtc tggccaccgg cttctacccc gaccacgtgg agctgtcctg gtgggtgaac 540

ggcaaggagg tgcacagcgg cgtgtctacc gacccccagc ccctgaagga gcagcccgcc 600

ctgaacgaca gccggtactg cctgtcctcc agactgagag tgagcgccac cttctggcag 660

aacccccgga accacttccg gtgccaggtg cagttctacg gcctgagcga gaacgacgag 720

tggacccagg accgggccaa gcccgtgacc cagattgtga gcgccgaggc ctggggcagg 780

gccgactgcg gcttcaccag cgagagctac cagcagggcg tgctgagcgc caccatcctg 840

tacgagatcc tgctgggcaa ggccaccctg tacgccgtgc tggtgtctgc cctggtgctg 900

atggctatgg tgaagcggaa ggacagccgg ggctaa 936

<210> 20
<211> 311
<212> PRT
<213> Artificial sequence

<220>
<223> beta chain amino acid sequence of TCR

<400> 20

Met Ser Ile Gly Leu Leu Cys Cys Ala Ala Leu Ser Leu Leu Trp Ala

```
1               5                   10                  15

Gly Pro Val Asn Ala Gly Val Thr Gln Thr Pro Lys Phe Gln Val Leu
            20                  25                  30

Lys Thr Gly Gln Ser Met Thr Leu Gln Cys Ala Gln Asp Met Asn His
        35                  40                  45

Glu Tyr Met Ser Trp Tyr Arg Gln Asp Pro Gly Met Gly Leu Arg Leu
    50                  55                  60

Ile His Tyr Ser Val Gly Ala Gln Thr Thr Asp Gln Gly Glu Val Pro
65                  70                  75                  80

Asn Gly Tyr Asn Val Ser Arg Ser Thr Ile Glu Asp Phe Pro Leu Arg
                85                  90                  95

Leu Leu Ser Ala Ala Pro Ser Gln Thr Ser Val Tyr Phe Cys Ala Ser
            100                 105                 110

Ser Tyr Leu Gly Asn Thr Gly Glu Leu Phe Phe Gly Glu Gly Ser Arg
        115                 120                 125

Leu Thr Val Leu Glu Asp Leu Lys Asn Val Phe Pro Pro Glu Val Ala
    130                 135                 140

Val Phe Glu Pro Ser Glu Ala Glu Ile Ser His Thr Gln Lys Ala Thr
145                 150                 155                 160

Leu Val Cys Leu Ala Thr Gly Phe Tyr Pro Asp His Val Glu Leu Ser
                165                 170                 175

Trp Trp Val Asn Gly Lys Glu Val His Ser Gly Val Ser Thr Asp Pro
            180                 185                 190

Gln Pro Leu Lys Glu Gln Pro Ala Leu Asn Asp Ser Arg Tyr Cys Leu
        195                 200                 205

Ser Ser Arg Leu Arg Val Ser Ala Thr Phe Trp Gln Asn Pro Arg Asn
    210                 215                 220

His Phe Arg Cys Gln Val Gln Phe Tyr Gly Leu Ser Glu Asn Asp Glu
225                 230                 235                 240

Trp Thr Gln Asp Arg Ala Lys Pro Val Thr Gln Ile Val Ser Ala Glu
                245                 250                 255
```

Ala Trp Gly Arg Ala Asp Cys Gly Phe Thr Ser Glu Ser Tyr Gln Gln
260 265 270

Gly Val Leu Ser Ala Thr Ile Leu Tyr Glu Ile Leu Leu Gly Lys Ala
275 280 285

Thr Leu Tyr Ala Val Leu Val Ser Ala Leu Val Leu Met Ala Met Val
290 295 300

Lys Arg Lys Asp Ser Arg Gly
305 310

<210> 21
<211> 822
<212> DNA
<213> Artificial sequence

<220>
<223> alpha chain nucleic acid sequence of TCR

<400> 21
atggagaccc tgctgggcct gctgatcctg tggctgcagc tccagtgggt gtccagcaag 60

caggaggtga cccagatccc tgccgccctg agcgtgcccg agggcgagaa cctggtgctg 120

aactgcagct tcaccgactc cgccatctac aacctgcagt ggttccggca ggaccccggc 180

aagggcctga ccagcctgct gctgattcaa tcctcgcagc gggagcagac cagcggacgg 240

ctgaacgcca gcctggacaa gagcagcggc cggagcaccc tgtacatcgc cgccagccag 300

cccggcgaca gcgccaccta cctgtgcgct gtgcggcctc tgctggacgg cacctacatc 360

cccaccttcg gcagaggcac cagcctgatc gtgcacccct acatccagaa ccccgacccc 420

gccgtgtacc agctgcggga cagcaagagc agcgacaagt ctgtgtgcct gttcaccgac 480

ttcgacagcc agaccaatgt gagccagagc aaggacagcg acgtgtacat caccgacaag 540

accgtgctgg acatgcggag catggacttc aagagcaaca gcgccgtggc ctggagcaac 600

aagagcgact tcgcctgcgc caacgccttc aacaacagca ttatccccga ggacaccttc 660

ttccccagcc ccgagagcag ctgcgacgtg aaactggtgg agaagagctt cgagaccgac 720

accaacctga acttccagaa cctgagcgtg atcggcttca gaatcctgct gctgaaggtg 780

gccggattca acctgctgat gaccctgcgg ctgtggagca gc 822

<210> 22
<211> 274
<212> PRT
<213> Artificial sequence

<220>
<223> alpha chain amino acid sequence of TCR

<400> 22

```
Met Glu Thr Leu Leu Gly Leu Leu Ile Leu Trp Leu Gln Leu Gln Trp
1               5                   10                  15

Val Ser Ser Lys Gln Glu Val Thr Gln Ile Pro Ala Ala Leu Ser Val
            20                  25                  30

Pro Glu Gly Glu Asn Leu Val Leu Asn Cys Ser Phe Thr Asp Ser Ala
        35                  40                  45

Ile Tyr Asn Leu Gln Trp Phe Arg Gln Asp Pro Gly Lys Gly Leu Thr
    50                  55                  60

Ser Leu Leu Leu Ile Gln Ser Ser Gln Arg Glu Gln Thr Ser Gly Arg
65                  70                  75                  80

Leu Asn Ala Ser Leu Asp Lys Ser Ser Gly Arg Ser Thr Leu Tyr Ile
                85                  90                  95

Ala Ala Ser Gln Pro Gly Asp Ser Ala Thr Tyr Leu Cys Ala Val Arg
            100                 105                 110

Pro Leu Leu Asp Gly Thr Tyr Ile Pro Thr Phe Gly Arg Gly Thr Ser
        115                 120                 125

Leu Ile Val His Pro Tyr Ile Gln Asn Pro Asp Pro Ala Val Tyr Gln
    130                 135                 140

Leu Arg Asp Ser Lys Ser Ser Asp Lys Ser Val Cys Leu Phe Thr Asp
145                 150                 155                 160

Phe Asp Ser Gln Thr Asn Val Ser Gln Ser Lys Asp Ser Asp Val Tyr
                165                 170                 175

Ile Thr Asp Lys Thr Val Leu Asp Met Arg Ser Met Asp Phe Lys Ser
            180                 185                 190

Asn Ser Ala Val Ala Trp Ser Asn Lys Ser Asp Phe Ala Cys Ala Asn
        195                 200                 205

Ala Phe Asn Asn Ser Ile Ile Pro Glu Asp Thr Phe Phe Pro Ser Pro
    210                 215                 220

Glu Ser Ser Cys Asp Val Lys Leu Val Glu Lys Ser Phe Glu Thr Asp
225                 230                 235                 240

Thr Asn Leu Asn Phe Gln Asn Leu Ser Val Ile Gly Phe Arg Ile Leu
                245                 250                 255
```

Leu Leu Lys Val Ala Gly Phe Asn Leu Leu Met Thr Leu Arg Leu Trp
260 265 270

Ser Ser

<210> 23
<211> 936
<212> DNA
<213> Artificial sequence

<220>
<223> beta chain nucleic acid sequence of TCR

<400> 23
atgagcatcg gcctgctgtg ctgcgccgcc ctgagcctgc tgtgggcagg acccgtgaac 60

gccggagtga cccagacccc caagttccag gtgctgaaaa ccggccagag catgaccctg 120

cagtgcgccc aggacatgaa ccacgagtac atgagctggt atcggcagga ccccggcatg 180

ggcctgcggc tgatccacta ctctgtgggc gccgggacca ccgaccgcgg cgaggtgccc 240

aacggctaca atgtgagccg gagcaccatc gaggacttcc ccctgcggct gctgagcgct 300

gcccccagcc agaccagcgt gtacttctgc gccagcagct atgtgggcga caccggcgag 360

ctgttcttcg gcgagggctc caggctgacc gtgctggagg acctgaagaa cgtgttcccc 420

cccgaggtgg ccgtgttcga gcccagcgag gccgagatca gccacaccca gaaggccaca 480

ctggtgtgtc tggccaccgg cttctacccc gaccacgtgg agctgtcctg gtgggtgaac 540

ggcaaggagg tgcacagcgg cgtgtctacc gacccccagc ccctgaagga gcagcccgcc 600

ctgaacgaca gccggtactg cctgtcctcc agactgagag tgagcgccac cttctggcag 660

aacccccgga accacttccg gtgccaggtg cagttctacg gcctgagcga gaacgacgag 720

tggacccagg accgggccaa gcccgtgacc cagattgtga gcgccgaggc ctggggcagg 780

gccgactgcg gcttcaccag cgagagctac cagcagggcg tgctgagcgc caccatcctg 840

tacgagatcc tgctgggcaa ggccaccctg tacgccgtgc tggtgtctgc cctggtgctg 900

atggctatgg tgaagcggaa ggacagccgg ggctaa 936

<210> 24
<211> 311
<212> PRT
<213> Artificial sequence

<220>
<223> beta chain amino acid sequence of TCR

<400> 24

Met Ser Ile Gly Leu Leu Cys Cys Ala Ala Leu Ser Leu Leu Trp Ala

```
1               5                   10                  15

Gly Pro Val Asn Ala Gly Val Thr Gln Thr Pro Lys Phe Gln Val Leu
            20                  25                  30

Lys Thr Gly Gln Ser Met Thr Leu Gln Cys Ala Gln Asp Met Asn His
        35                  40                  45

Glu Tyr Met Ser Trp Tyr Arg Gln Asp Pro Gly Met Gly Leu Arg Leu
    50                  55                  60

Ile His Tyr Ser Val Gly Ala Gly Thr Thr Asp Arg Gly Glu Val Pro
65                  70                  75                  80

Asn Gly Tyr Asn Val Ser Arg Ser Thr Ile Glu Asp Phe Pro Leu Arg
                85                  90                  95

Leu Leu Ser Ala Ala Pro Ser Gln Thr Ser Val Tyr Phe Cys Ala Ser
            100                 105                 110

Ser Tyr Val Gly Asp Thr Gly Glu Leu Phe Phe Gly Glu Gly Ser Arg
        115                 120                 125

Leu Thr Val Leu Glu Asp Leu Lys Asn Val Phe Pro Pro Glu Val Ala
    130                 135                 140

Val Phe Glu Pro Ser Glu Ala Glu Ile Ser His Thr Gln Lys Ala Thr
145                 150                 155                 160

Leu Val Cys Leu Ala Thr Gly Phe Tyr Pro Asp His Val Glu Leu Ser
                165                 170                 175

Trp Trp Val Asn Gly Lys Glu Val His Ser Gly Val Ser Thr Asp Pro
            180                 185                 190

Gln Pro Leu Lys Glu Gln Pro Ala Leu Asn Asp Ser Arg Tyr Cys Leu
        195                 200                 205

Ser Ser Arg Leu Arg Val Ser Ala Thr Phe Trp Gln Asn Pro Arg Asn
    210                 215                 220

His Phe Arg Cys Gln Val Gln Phe Tyr Gly Leu Ser Glu Asn Asp Glu
225                 230                 235                 240

Trp Thr Gln Asp Arg Ala Lys Pro Val Thr Gln Ile Val Ser Ala Glu
                245                 250                 255
```

```
Ala Trp Gly Arg Ala Asp Cys Gly Phe Thr Ser Glu Ser Tyr Gln Gln
            260                 265                 270

Gly Val Leu Ser Ala Thr Ile Leu Tyr Glu Ile Leu Leu Gly Lys Ala
        275                 280                 285

Thr Leu Tyr Ala Val Leu Val Ser Ala Leu Val Leu Met Ala Met Val
    290                 295                 300

Lys Arg Lys Asp Ser Arg Gly
305                 310
```